# EUROPEAN PATENT APPLICATION

(11) **EP 2 305 242 A2**
(43) Date of publication of application: **06.04.2011**
(21) Application number: 10190258.3
(22) Date of filing: 03.11.2006
(51) Int. Cl.: A61K 31/202, A61K 38/44, C12N 9/02, A61K 38/16, C07K 16/18, C12N 1/00, C12N 5/16, C12Q 1/68, C12N 5/10, G01N 33/48

(54) **20-carboxy-(S)-trioxilin A3 for treating neurologic disorders**

(30) Priority: 04.11.2005 EP 05292341
(62) Divisional of application: 06819241.8
(71) Applicant: Commissariat à l'Énergie Atomique et aux Énergies Alternatives, 75015 Paris (FR)
(72) Inventor: Fischer, Judith, 75005, Paris (FR)
(74) Representative: Starck-Loudes, Anne-Caroline

(57) **Abstract**

The present application relates to compositions and methods for treating neurologic disorders. The present application more particularly discloses the identification of new genes and metabolic pathways involved in neurologic disorders, which provide novel targets and approaches for treating said disorders and for screening biologically active compounds. The present invention also provides various products and constructs, such as probes, primers, vectors, recombinant cells, which can be used to implement the above methods. The invention may be used to detect or treat various neurologic disorders, in various subjects, including mammalian subjects, particularly human beings.

## Description

The present application relates to compositions and methods for treating skin disorders. The present application more particularly discloses the identification of new genes and metabolic pathways involved in ichthyosis and, more generally, skin hydration and protection of skin in inflammatory events, which provide novel targets and approaches for treating said disorders and for screening biologically active compounds. The present invention also provides various products and constructs, such as probes, primers, vectors and recombinant cells, which can be used to implement the above methods. The invention may be used to detect or treat various skin disorders, particularly dry skin disorders and inflammatory events, in various subjects, including mammalian subjects, particularly human beings.

The metabolic pathways from arachidonic acid ("AA"), which lead to leukotrienes, hepoxilins and lipoxins, have been mainly defined by the biochemical identification of their numerous intermediates and products.

Leukotrienes and lipoxins are potent lipid mediators derived from AA, via activation of 5-lipoxygenase and 15-lipoxygenase enzymes. All of them belong to the large eicosanoid family of small lipid messengers generated by divergent metabolic pathways from AA, and are implicated in biological functions as diverse as chemotaxis, vascular permeability, smooth muscle contraction, inflammation, and in the pathophysiology of various inflammatory and hypersensitivity disorders such as asthma. Recent reviews have summarized the present knowledge of their metabolism, biological actions, and implication in common and rare disorders (21).

Another metabolic pathway, which also starts from AA but proceeds via activation of 12-lipoxygenase, leads to several trihydroxytetraene products of the hepoxilin family (acronym for hydroxy epoxid with a biological activity in insulin secretion). Hepoxilin and other intermediates or products act primarily on calcium and potassium channels in membranes, which ultimately result in a wide range of functions including insulin secretion, vascular permeability, vasoconstriction, synaptic transmission, cell volume regulation, and activation of neutrophils and platelets (18,19). However, paradoxically, this pathway has received less attention since its discovery during the eighties.

The present inventors now disclose a novel gene from the metabolic pathway leading to derivatives of hepoxilin with 12(R)-chirality, as well as its unexpected and undisclosed implication in skin disorders. The present application thus provides novel genes, metabolites, targets and therapeutic approaches to the treatment or prevention of particular skin conditions.

More particularly, the present invention reports the identification of mutations in a non-syndromic autosomal recessive congenital ichthyosis (ARCI), in a new gene mapping within a previously identified locus on chromosome 19p12-q12 which has been defined as LI3 in the OMIM database (MIM 604777). Seven homozygous mutations, including five missense mutations and two deletions, were identified in a new gene, FLJ39501, on chromosome 19p12 in 21 patients from 12 consanguineous families from Algeria, France, Italy and Lebanon. FLJ39501 encodes a protein which was found to be a cytochrome P450, family 2, subfamily E, polypeptide 2 homolog of the leukotriene B4-ω-hydroxylase (CYP4F2), and could catalyze the 20-hydroxylation of trioxilin A3 from the 12(R)-lipoxygenase pathway. Further oxidation of this substrate by the fatty alcohol:nicotinamide-adenine dinucleotide oxidoreductase (FAO) enzyme complex, in which one component, ALDH3A2, is known to be mutated in Sjögren-Larsson syndrome (characterized by ichthyosis and spastic paraplegia) (MIM 270200) (28-30), would lead to 20-carboxy-(R)-trioxilin A3.

Based on the results presented here, it can be proposed that 20-carboxy-(R)-trioxilin A3 is involved in skin hydration and represents the essential missing product in most forms of ARCI. Its chiral homolog, 20-carboxy-(S)-trioxilin A3, could be implicated in spastic paraplegia and in the maintenance of neuronal integrity.

A particular object of this invention thus resides in a method of detecting, diagnosing or characterizing the presence of, or predisposition to a skin disorder in a subject, comprising assessing, in a biological sample from said subject, the presence of a genetic alteration in the FLJ39501 gene or corresponding protein, the presence of a genetic alteration in said gene or protein being indicative of the presence of or predisposition to a skin disorder in said subject.

As will be disclosed further below, the genetic alteration may be a mutation, a deletion, an insertion, a splice site mutation, an inversion, an addition and/or a substitution of one or more residues in said gene or encoded protein, typically a mutation. The biological sample may be any tissue, cell or fluid that contains a FLJ39501 gene or polypeptide, preferably a sample comprising genomic DNA from the subject.

A further aspect of this invention resides in nucleic acid probes and primers that can specifically hybridise to or amplify all or a distinctive part of the FLJ39501 gene.

A further aspect of this invention is a method of amplifying a FLJ39501 gene, the method comprising:
(i) providing a biological sample containing a FLJ39501 gene or a portion thereof,
(ii) contacting said sample with (a pair of) primers as defined above, and
(iii) amplifying the FLJ39501 gene or mRNA.

Another object of this invention is a method of selecting or identifying biologically active compounds, comprising contacting a candidate compound with a FLJ39501 protein, and determining whether said compound binds to or modulate the activity of said protein. In a particular embodiment, the method comprises contacting the test compound with a recombinant host cell expressing a FLJ39501 protein, and selecting the compounds that bind said protein or modulate its activity.

A further object of this invention is a recombinant host cell comprising a genetic construct encoding a FLJ39501 protein, said cell expressing said protein. The recombinant cell of this invention may be a prokaryotic or eukaryotic cell. It may be a primary cell or an established cell line, of various origins.

Still a further aspect of this invention resides in an antibody that specifically binds a FLJ39501 protein.

An other object of this invention resides in the use of a compound that modulates FLJ39501 activity for the manufacture of a medication (or medicament) for the treatment of a skin disorder, as well as in a corresponding method of treatment. The compound preferably stimulates or mimics the activity of a FLJ39501 protein. The compound may be identified using a method as described above. The compound may be a FLJ39501 protein or a corresponding nucleic acid.

A further object of this invention resides in the use of 20-carboxy-(R)-trioxilin A3, an analog thereof or a pharmaceutically acceptable salt or hydrate thereof, for the manufacture of a medication (or medicament) for the treatment of a skin disorder, particularly for skin hydration, as well as in a corresponding method of treatment.

A further object of this invention resides in the use of 20-carboxy-(S)-trioxilin A3, an analog thereof or a pharmaceutically acceptable salt or hydrate thereof, for the manufacture of a medication (or medicament) for the treatment of a neurologic disorder , as well as in a corresponding method of treatment.

An other object of this invention is a composition comprising 20-carboxy-(R)-trioxilin A3, an analog thereof or a pharmaceutically acceptable salt or hydrate thereof, and a pharmaceutically acceptable carrier, preferably for topical application.

Still another object of this invention is 20-carboxy-(R)-trioxilin A3, an analog thereof or a pharmaceutically acceptable salt or hydrate thereof, as a medicament, particularly for the treatment of dry skin.

A further object of this invention is a method of screening or identifying or isolating a receptor of 20-carboxy-(S)-trioxilin A3 or of 20-carboxy-(R)-trioxilin A3, comprising contacting said compound with a candidate sample and determining whether the compound binds said material, preferably in a specific manner. Specific binding designates an affinity above the background binding to other sample that does not contain a receptor. Specific binding may be determined by assessing the binding under different conditions or by comparing the binding to the binding with a reference sample that does not contain a receptor. Binding may be assessed using a number of techniques known per se in the art, such as the use of a labelled compound.

A further aspect of this invention is a method of selecting or identifying an agonist of 20-carboxy-(S)-trioxilin A3 or of 20-carboxy-(R)-trioxilin A3, the method comprising contacting a candidate sample suspected to contain such an agonist with a sample comprising a receptor for 20-carboxy-(S)-trioxilin A3 or 20-carboxy-(R)-trioxilin A3, and determining whether any binding occurs. Preferably, binding is carried out in the presence of 20-carboxy-(S)-trioxilin A3 or of 20-carboxy-(R)-trioxilin A3, and the method comprises determining whether the candidate sample competes with said compound for binding to the receptor.

The invention may be used to detect, diagnose, classify, treat (including the prevention of) a skin disorder in a subject, particularly a dry skin disorder, notably dermatosis, such as atopic skin, contact dermatitis, eczema, psoriasis, or a keratinization disorder such as an ichthyosis and a palmoplantar keratoderma, or to protect skin in inflammatory events; or to screen, identify, select or produce compounds for use in treating or preventing a skin disorder.

### DEFINITIONS

Dry skin disorders: Within the context of the present invention, a "dry skin" disorder refers generally to any pathology associated with or resulting from an impaired skin barrier function and/or with epidermis dryness. Dryness and skin barrier disorders are not a single entity, but are characterized by differences in chemistry and morphology in the epidermis. (1,2,3). Specific examples of such diseases or disorders include atopic skin, contact dermatitis, eczema, psoriasis and keratinisation disorders such as ichthyosis. Skin disorders also include skin inflammation as well as skin affections resulting from various type of aggressions, including chemical (acid, sodium hydroxide, etc.), physical (e.g., X-ray, UV, etc.), or biological (e.g., infections) aggressions.

Neurologic disorder: Within the context of the present invention, a "neurologic" disorder refers generally to any disorder of the peripheral or central nervous system, including degenerative disorders, particularly spastic paraplegia, multiple sclerosis and amyotrophic lateral sclerosis. The term also includes the maintenance or neuronal integrity.

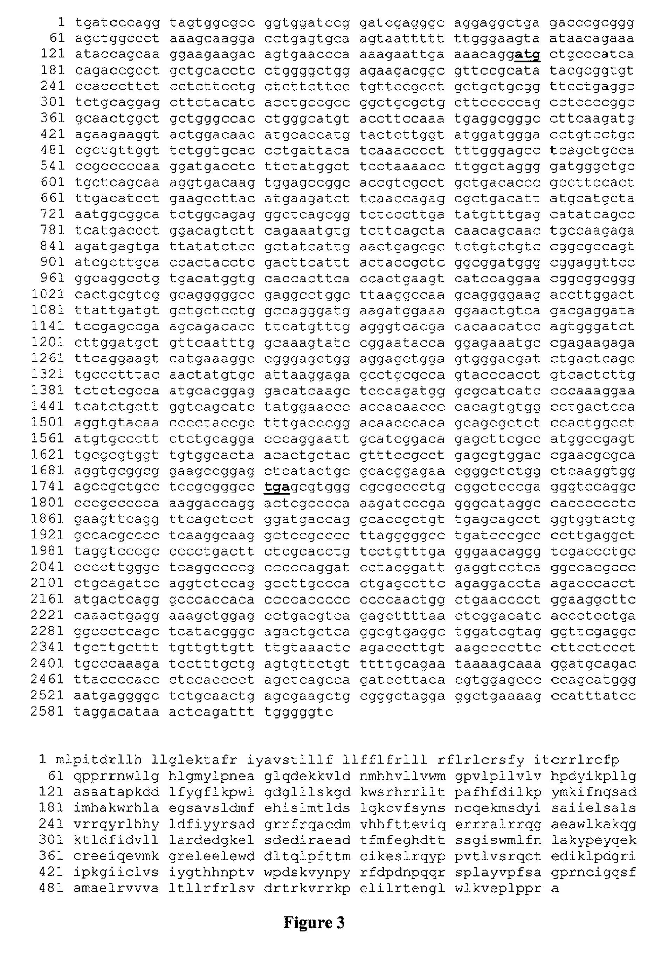

FLJ39501 gene: Within the context of the present invention, the term FLJ39501 gene refers to a nucleic acid molecule encoding a cytochrome P450, family 2, subfamily E, polypeptide 2 homolog of the leukotriene B4-ω-hydroxylase. A particular FLJ39501 gene is a nucleic acid molecule comprising a sequence as disclosed in Figure 3 (SEQ ID No: 1), as well as any portion thereof, naturally occurring variant thereof, such as variants resulting from polymorphisms, splicings, etc., and orthologs thereof. The term " FLJ39501 gene" applies to coding and non-coding parts of the genomic DNA or RNA, such as promoter, poly A, intronic sequences, etc. The term gene also includes the coding region, in the form of genomic DNA, cDNA, RNA (pre-rRNA, messenger RNA, etc), etc. or any synthetic nucleic acid comprising all or part of the sequence thereof. Synthetic nucleic acid includes cDNA, prepared from RNAs, and containing at least a portion of a sequence of the FLJ39501 gene, such as for example one or more introns or a portion containing one or more mutations. Those nucleic acids can be isolated from known banks by hybridization techniques under stringent conditions at best. They can also be genetically or chemically synthesized.

The sequence of wild type human FLJ39501 gene is available in the literature under the following accession numbers : *FLJ39501*: GeneID 126410; UniGene Hs.156452; mRNA: NM_173483 (GI:42657191); AK096820 (GI:21756394).

A portion or part of a gene means at least 3 nucleotides, preferably at least 9 nucleotides, even more preferably at least 15 nucleotides, and can contain as many as 1000 nucleotides or more. Such a portion can be obtained by any technique well known in the art, e.g., enzymatic cleavage, chemical synthesis or a combination thereof. A distinctive portion of a FLJ39501 gene refers to a fragment of at least 5, preferably at least 8 consecutive nucleotides that are specific for a sequence as disclosed above. A distinctive portion may also contain a genetic alteration, as disclosed below.

FLJ39501 protein: Any polypeptide encoded by a FLJ39501 gene as defined above. As a specific example, the sequence of wild type human FLJ39501 protein is available in the literature under the following accession number: NP_775754 (GI:27735073). A preferred FLJ39501 protein is a polypeptide that catalyzes the 20-hydroxylation of trioxilin A3 from the 12(R)-lipoxygenase pathway. The term FLJ39501 protein includes any portion, fragment, derivative or mutants of a wild type human FLJ39501 protein as defined above. A specific example of a human FLJ39501 protein comprises SEQ ID NO: 2 (Figure 3). A portion or fragment of a protein typically comprises at least 5, 8, 10, 15, 20 or 25 consecutive amino acid residues thereof.

### IDENTIFICATION OF A NOVEL GENE INVOLVED IN DRY SKIN DISORDERS

Autosomal recessive congenital ichthyoses (ARCI) are a clinically and genetically heterogeneous group of diseases, characterized by generalized desquamation of the skin, usually with erythema (1-3). Most of the patients are born as collodion babies. The disease may progress to two main clinical forms, either lamellar ichthyosis (LI) or nonbullous congenital ichthyosiform erythroderma (NCIE). The estimated incidence is between one in 300,000 and one in 500,000 for both forms. In LI, the scales are large, adherent, dark and pigmented without skin erythema, whereas in NCIE, the scales are fine and white on an erythematous background, although they are larger and grayish on the limbs (1,4). Overlapping phenotypes have been described, and may depend on the age of the patient, and the region of the body. LI is characterized histologically by orthohyperkeratosis and mild focal parakeratosis. Hyperkeratosis associated with an increase in stratum corneum thickness, a normal or prominent granular layer, and increased mitoses suggest a hyperproliferative epidermal defect in NCIE (1-6). Prominent dermal flood vessels and an upper lymphocytic infiltrate may explain the erythroderma. The terminal differentiation of the epidermis is disturbed in both forms, leading to a reduced barrier function and defects in the stratum corneum lipid composition (1-7).

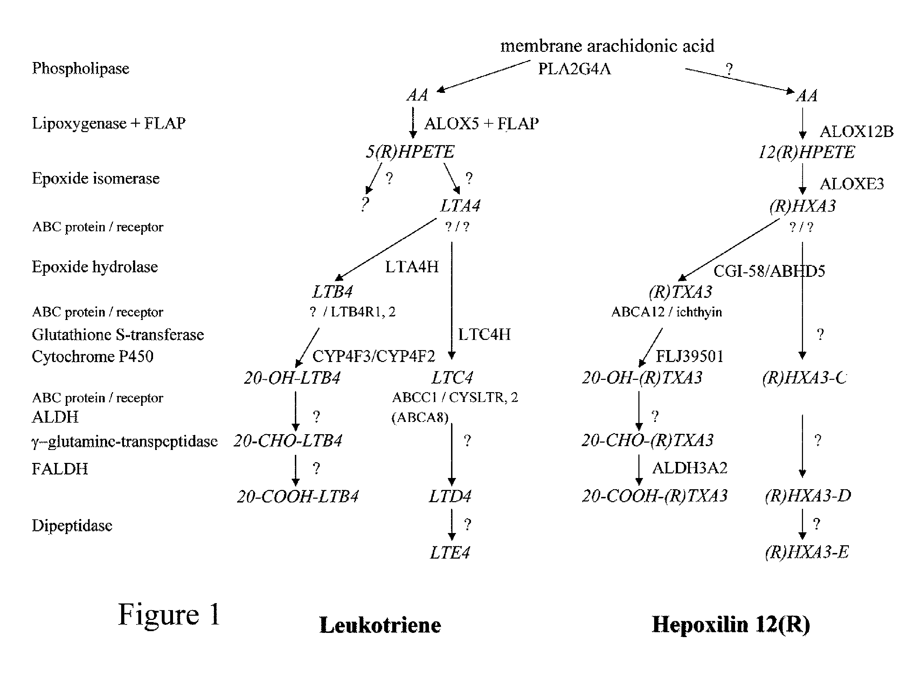

Several defective genes have been identified in ARCI (8-14). Some of them govern the synthesis of enzymes and transporters directly involved in the production, transport or assembly of components of the stratum corneum. For instance, TGM1 encodes a transglutaminase implicated in the cross-linking of structural proteins (involucrin, loricrin) (8,9,15), ABCA12 is suspected to be a lipid transporter (11,16), and STS, a cholesterol sulfatase, is implicated in the metabolism of cholesterol sulfate (17). Two other genes, which have found to be defective in ARCI, belong to a metabolic pathway starting from arachidonic acid and leading to compounds of the 12-lipoxygenase family (Figure 1) (21). As we observed mutations in two physically linked genes, ALOX12B and ALOXE3, in patients with ARCI with the same phenotype, we proposed that the product of one enzyme could be the substrate of the other (10). The lipoxygenase ALOX12B and the hydroperoxide isomerase ALOXE3 catalyze the successive steps of the 12(R)-lipoxygenase pathway, which begins with arachidonic acid and leads to 12(R)-hepoxilin A3 [12(R)-HXA3] via 12(R)-hydroperoxyeicosatetraenoic acid [12(R)-HPETE] (18). Since we observed mutations in CGI-58/ABHD5 in Chanarin-Dorfinan syndrome (13), in which an ichthyosis is associated with a nonlysosomal multisystem triglyceride storage disease, and since this protein could be an epoxide hydrolase, we suggested that the lack of the terminal product of the 12(R)-lipoxygenase pathway, 12(R)-TXA3, underlies most forms of ARCI (12). Surprisingly, the homolog, 12(S)-TXA3 in the 12(S)-lipoxygenase pathway, was considered to be inactive (22,23), in contrast with other epoxide hydrolysis products, such as LTB4 in the leukotriene pathway or trioxilin C3 in the hepoxilin pathways, which are biologically active (24-26). We have proposed that two other genes could also belong to the same pathway: (1) ABCA12, which might be an export carrier of this mediator into the extracellular space; and (2) ichthyin, that we disclosed to be a receptor for 12(R)-TXA3 (12).

We had previously localized another ichthyosis gene on chromosome 19p12-q12 between the markers D19S899 (AFMb021yb9) and D19S405 (AFM205yf10) in six consanguineous families with ARCI by homozygosity mapping (27). Other families, mainly from Algeria, were also found to have ichthyosis linked to the chromosomal region on 19p12-q12. We refined the linkage interval to 3.06 Mb by dense microsatellite genotyping based on recombination events. Weak linkage disequilibrium was detected for the marker GATA27C12. Twenty-eight candidate genes in the interval were excluded before we identified deleterious mutations in a new gene FLJ39501. This gene is annotated as a homolog of the cytochrome P450, family 2, subfamily E, polypeptide 2 (CYP4F2). The present study and the previous implication of a defective aldehyde dehydrogenase (ALDH3A2) in Sjögren-Larsson syndrome (ichthyosis and spastic paraplegia, MIM 270200) (28-30) now imply that further derivatives of the 12(R)-lipoxygenase pathway, through ω-hydroxylation, could be the missing products in ichthyosis.

The identification of this novel metabolic pathway, including the FLJ39501 protein, offers strong opportunities to design and provide simple treatments to patients. In particular, the terminal product of this pathway, 20-carboxy-(R)-trioxilin A3, is easily accessible and enables the design and the synthesis of analogues, agonists and antagonists thereof, with therapeutic utility. This compound and the FLJ39501 protein/gene also allow the design of effective screening or diagnosis methods, e.g., to characterize the corresponding receptor, which could be the ichthyin protein previously identified in another form of ARCI (12), and to develop new drug candidates and detect skin disorders, respectively.

### METHODS OF DIAGNOSING SKIN DISORDERS

The present invention discloses that the FLJ39501 gene or encoded polypeptide is genetically altered in patients having (or at risk of developing) skin disorders. This gene and polypeptide thus represent valuable biomarkers, suitable for monitoring predisposition, presence or progression of a skin disorder in a subject.

In this respect, a particular object of this invention resides in a method of detecting, diagnosing or characterizing the presence of, or predisposition to a skin disorder in a subject, comprising assessing, in a biological sample from said subject, the presence of a genetic alteration in the FLJ39501 gene or corresponding protein, the presence of a genetic alteration in said gene or protein being indicative of the presence of or predisposition to a skin disorder in said subject.

The alteration in the FLJ39501 gene or polypeptide may be any genetic alteration, such as a mutation, a deletion, an inversion, an insertion, a splice site mutation, an addition and/or a substitution of one or more residues in said gene or encoded polypeptide. Preferred genetic alterations are exon deletions, single nucleotide deletions and mutations in a coding or non-coding region, particularly mutations in a coding region leading to a change in amino acid sequence in the encoded polypeptide, e.g., an amino acid substitution, a frameshift and/or a truncated polypeptide sequence. Such alterations may affect any one of exons 1-12, for instance exons 1, 6, 7, 8 and 10. Specific examples of genetic alterations in the FLJ39501 gene are disclosed in Table 1, and include a large homozygous deletion in which exons 3-12 were missing (F3), another one was a small deletion of one bp (980delC) in exon 7 (F9), which leads to a frameshift and a stop codon, and five were missense mutations: 177C→G (F59L) in exon 1 (F5); 728G→A (R243H) in exon 6 (F4); 1114C→T (R372W) in exon 8 (F7); and 1303C→T (H435Y) (F1, F2, F6, F8, F10, and F11), and 1306C→G (H436D) in exon 10 (F12). The same 1303C→T (H435Y) mutation was shared by six families, all of which were from Algeria with the exception of family F1 which had French origins. The numbering of nucleotides corresponds to the coding region only, which starts with 1 from the A of the ATG codon in position 168 of SEQ ID NO: 1, and ends up with TGA codon at position 1763 (see Figure 3, bold characters).

The presently identified missense mutations were all situated in parts of the gene that are highly conserved between mice, rats and humans. None of these sequence variations were found in 100 normal chromosomes from a Mediterranean control population.

The methods comprise, typically, detection of the presence of a mutation in a FLJ39501 gene or polypeptide in a biological sample from a subject. The biological sample comprises any material such as cells, a tissue, an organ, a fluid, etc or fractions thereof, which contain nucleic acids or polypeptides. Specific examples of such biological samples include white blood cells or fluids, such as blood, plasma or urine, biopsies, skin and mucosa, which may possibly be obtained by non-invasive methods or from tissue collections, if necessary. Furthermore, since the FLJ39501 gene is found within the cells, tissues or fluids mentioned above, the sample is usually treated to render the gene available for detection or analysis. Treatment may comprise any conventional fixation technique, cell lysis (mechanical or chemical or physical), or any other conventional method used in immuno-histology or biology, for instance.

In a first variant, the present invention provides a method of detecting the presence of an altered FLJ39501 polypeptide. This can be determined by any suitable technique known to the skilled artisan, including by immuno-assay (ELISA, EIA, RIA, etc.). This can be made using any affinity reagent specific for a FLJ39501 polypeptide, more preferably any antibody or fragment or derivative thereof, particularly any affinity reagent specific for an altered FLJ39501 polypeptide. In a particular embodiment, the FLJ39501 polypeptide is detected with an anti-FLJ39501 antibody (or a fragment thereof), more preferably a monoclonal antibody, as described above. The antibody (or affinity reagent) may be labelled by any suitable method (radioactivity, fluorescence, enzymatic, chemical, etc). Alternatively, FLJ39501-antibody immune complexes may be revealed (and/or quantified) using a second reagent (e.g., antibody), labelled, that binds to the anti-FLJ39501 antibody, for instance.

In a second variant of the present invention, the presence of an altered FLJ39501 gene is detected. This can be done using various techniques, as described below.

In a particular embodiment, the method comprises the characterization of all or part of a FLJ39501 gene in the sample and the comparison of said gene to the wild-type FLJ39501 gene. Comparison can be made by (partial) sequencing, gel migration, hybridization, etc.

In another particular embodiment, the method comprises detecting all or part of a FLJ39501 gene in the sample by selective hybridisation to a specific nucleic acid probe.

Another method of the invention comprises the amplification of a FLJ39501 gene or a portion thereof, and the determination of the presence of an alteration in the amplification product.

In particular embodiments, FLJ39501 gene alterations are assessed by quantitative RT-PCR, LCR (Ligase Chain Reaction), TMA (Transcription Mediated Amplification), PCE (an enzyme amplified immunoassay) or bDNA (branched DNA signal amplification) assays.

In a particular embodiment, a FLJ39501 gene alteration is determined by *in vitro* or *ex vivo* cDNA synthesis, (PCR) amplification with FLJ39501-specific oligonucleotide primers, and analysis of PCR products.

RT-PCR amplification of a FLJ39501 mRNAs or gene may be performed using any pair of FLJ39501-specific primers. In particular any primers can be designed by the skilled artisan, such as any fragment of a FLJ39501 gene, for use in the amplification step and especially a pair of primers comprising a forward sequence and a reverse sequence wherein said primers of said pair hybridize with a region of a FLJ39501 gene (or flanking a FLJ39501 gene) and allow amplification of at least a portion of the FLJ39501 gene or of a portion of the FLJ39501 gene containing a genetic alteration. In a particular embodiment, the FLJ39501 cDNA is prepared using a conventional protocol with any primer disclosed in Table 2 (SEQ ID NOs: 3 to 30). Preferably, the FLJ39501 gene is amplified using any pair of primer as disclosed in Table 2.

Primers of this invention more preferably contain less than about 50 nucleotides even more preferably less than 30 nucleotides, typically less than about 25 or 20 nucleotides. Also, preferred primers usually contain at least 5, preferably at least 8 nucleotides, to ensure specificity. The sequence of the primer can be prepared based on the sequence of a FLJ39501 gene, to allow full complementarity therewith, preferably. Specific examples of primers of this invention are oligonucleotides comprising a sequence selected from SEQ ID Nos: 3-30.

Nucleic acid probes of this invention comprise (or specifically hybridise to) all or a distinctive part of the nucleic acid sequence of the FLJ39501 gene, preferably at least a distinctive part thereof, i.e., a portion comprising at least one of the above-mentioned mutations. The probes may comprise between 8 and 1000 nucleotides, or even more (e.g., the entire sequence of the gene). The probes are most preferably single stranded. The probes may be labeled using any known technique such as radioactivity, fluorescence, enzymatic, chemical, etc. This labeling can use for example Phosphor 32, biotin (16-dUTP), digoxygenin (11-dUTP).

It should be understood that the present invention shall not be bound or limited by particular detection or labeling techniques, which can essentially be applied to the FLJ39501 gene using ordinary skills.

The above methods can be implemented e.g., in vitro or ex vivo, in any suitable device or container or support, such as a tube, flask, plate, chip, column, etc.

The invention also relates to kits for implementing the above methods comprising at least a primer or a probe specific for a FLJ39501 gene and, optionally, reagents for a nucleic acid amplification or hybridization reaction. The reagents may include antibodies, probes, primers, devices, supports, etc.

The invention also relates to a nucleic acid comprising a mutated FLJ39501 gene sequence or a distinctive portion thereof.

As shown in the example, the invention now demonstrates a correlation between the presence of an alteration in a FLJ39501 gene in a subject and the presence, development or predisposition to a skin disorder.

### METHODS OF SCREENING BIOLOGICALLY ACTIVE COMPOUNDS

In a further aspect, the present invention also relates to the use of a FLJ39501 gene or polypeptide as a target for screening biologically active agents, particularly compounds active on dry skin disorders and/or on the hepoxilin pathway.

A particular object of this invention lies in methods of (in vitro, ex vivo or in vivo) selecting or identifying biologically active compounds, comprising contacting a candidate compound with a FLJ39501 protein and determining whether said compound binds to or modulate the activity of said protein. Binding may be determined by any technique known per se in the art, including ligand displacement, competition assays, direct binding using labeled compounds, immuno-precipitation, gel migration, etc. Modulation of the activity may be determined by assessing any conformational change, or by determining or measuring any secondary signal mediated by FLJ30501. The method may be carried out using FLJ39501 or a fragment of thereof (for instance the substrate-binding domain), which may be in isolated form, immobilized on a support, expressed in a lipid membrane or by an intact cell.

In a particular embodiment, the method comprises contacting a test compound with a recombinant host cell expressing a FLJ39501 protein or a fragment thereof, typically a substrate-binding fragment thereof, and selecting the compounds that bind said protein or modulates its activity. Such recombinant cells, which also represent particular objects of the present invention, may be any cell comprising a genetic construct encoding a FLJ39501 protein, said cell expressing said protein. The recombinant cell may be a prokaryotic cell or a eukaryotic cell. Examples of prokaryotic cells include bacterial cells, particularly E. coli. Eukaryotic cells include yeast cells (e.g., saccharomyces, Kluyveromyces, etc), mammalian cells, plant cells, insect cells, etc. Particular examples of mammalian cells include primary or established cell cultures from various species, including rodents, canine, equine, bovine, ovine, human, etc, and from various tissue cell type (e.g., keratinocytes, fibroblasts, hepatocytes, muscle cells, nervous cells, kidney cells, ovary cells, etc).

The recombinant cells may be prepared by conventional recombinant techniques, e.g., by introduction into a selected cell type of a genetic construct encoding a FLJ39501 protein, under conditions allowing expression of said protein. The genetic construct may be any plasmid, cosmid, artificial chromosome, virus, phage, episome, etc, which may be prepared according to techniques known in the art. The construct typically comprises, upstream from the coding sequence, a promoter region that causes expression of FLJ39501 in the selected cell. The construct may also include an origin of replication, or a marker gene, or a homologous region (allowing site-specific integration into the cell's genome), an integrase, etc. The construct may be introduced into the cells (or their ancestors) by techniques known per se in the art, such as electroporation, calcium-phosphate precipitation, conjugation, naked DNA transfer, transfection, infection, etc. Introduction may be performed in the presence of facilitating agents, such as liposomes, cationic lipids, polymers, etc. The recombinant cells may be selected and cultivated under conventional conditions. Cell expression of FLJ39501 may be verified by any binding assay, for instance in the presence of an antibody or ligand.

In this regard, a particular object of this invention is a genetic or nucleic acid construct comprising a nucleic acid sequence encoding a FLJ39501 protein, under the control of a promoter, preferably a heterologous promoter. The heterologous promoter may be any promoter that does not control FLJ39501 expression in a naturally occurring cell. Examples of such promoters include viral promoters (e.g., CMV, LTR, TK, SV40, etc), cell promoters (PGK, HAS, etc), bacterial promoters (Trp, Lac, etc), yeast promoters, as well as any artificial or chimeric promoter sequence. The construct may be incorporated into a vector as described above.

An other particular aspect of this invention resides in a method of selecting or identifying a compound that modulates the arachidonic acid metabolism, comprising contacting a candidate compound with a recombinant host cell expressing a FLJ39501 protein or a substrate-binding fragment or sub-unit thereof, and selecting the compounds that bind said protein or modulate its activity.

Preferred compounds of this invention are selected for their ability to bind the above proteins and to behave as agonists of the corresponding ligands, i.e., to mimic or stimulate the FLJ39501 activity.

Other methods of this invention comprise contacting a candidate molecule with a gene encoding said proteins, and selecting the molecules that bind to said gene or modulate the expression of said proteins. Particular molecules are those, which stimulate expression of said genes, particularly those that stimulate the transcriptional promoters of said genes.

Within the context of this invention, various candidate compounds may be tested in parallel, using different assay formats (microtitration plate, etc). The compound may be contacted with the cells for a sufficient period of time to allow binding to occur, and the binding or activity may be assessed as disclosed above. The invention is particularly suited to screen agonists of the FLJ39501 protein, or activators thereof.

### ANTIBODIES

Another aspect of this invention relates to an antibody that binds a FLJ39501 protein. The antibody may be a polyclonal or a monoclonal antibody. Furthermore, the term antibody also includes fragments and derivatives thereof, in particular fragments and derivatives of said monoclonal or polyclonal antibodies having substantially the same antigenic specificity. These include antibody fragments (e.g., Fab, Fab'2, CDRs, etc), humanized antibodies, poly-functional antibodies, Single Chain antibodies (ScFv), etc. These may be produced according to conventional methods, including immunization of an animal and collection of serum (polyclonal) or spleen cells (to produce hybridomas by fusion with appropriate cell lines).

To produce polyclonal antibodies from various species, the antigen is generally combined with an adjuvant (e.g., Freund's adjuvant) and administered to an animal, typically by subcutaneous injection. Repeated injections may be performed. Blood samples are collected and immunoglobulins or serum are separated. Monoclonal antibodies may be produced from various species as described for instance in Harlow et al (Antibodies: A laboratory Manual, CSH Press, 1988). Briefly, these methods comprise immunizing an animal with the antigen, followed by a recovery of spleen cells, which are then fused with immortalized cells, such as myeloma cells. The resulting hybridomas produce the monoclonal antibodies and can be selected by limit dilutions to isolate individual clones. Antibodies may also be produced by selection of combinatorial libraries of immunoglobulins, as disclosed for instance in Ward et al (Nature 341 (1989) 544).

Fab or F(ab')2 fragments may be produced by protease digestion, according to conventional techniques. Humanized antibodies can be prepared as previously described (Jones 1986; Riechmann 1988).

Preferred antibodies of this invention are prepared by immunization with a fragment of a FLJ39501 protein, preferably with an immunogenic fragment thereof, e.g., a fragment comprising at least an epitope, preferably of at least 5 amino acids.

Other preferred antibodies are monoclonal antibodies that specifically bind an altered FLJ39501 polypeptide, particularly a mutated FLJ39501 polypeptide.

The antibodies may be coupled to heterologous moieties, such as toxins, labels, drugs or other therapeutic agents, covalently or not, either directly or through the use of coupling agents or linkers.

The antibodies of this invention have various applications, including therapeutic, diagnostic, purification, detection, prophylactic, etc. *In vitro,* they can be used as screening agents or to purify the antigen from biological samples. They can also be used to detect or quantify the presence (or amounts) of a FLJ39501 polypeptide in a sample collected from a subject, typically a blood sample from a mammalian, specifically a human subject. Antibodies of this invention may also be used as agonists or antagonists of the FLJ39501 protein, particularly in a therapeutic context.

### NOVEL COMPOUNDS AND METHODS FOR TREATING SKIN DISORDERS

As mentioned above, the present invention discloses a novel gene in the metabolic pathway leading to derivatives of hepoxilin with (R)-chirality, as well as its unexpected and undisclosed implication in skin disorders. The present application allows the design of novel therapeutic approaches to the treatment or prevention of particular skin conditions, using compounds from this metabolic pathway, as well as analogs or agonists thereof, or compounds that regulate the expression of said gene.

A particular object of this invention more specifically resides in the use of a compound that modulates the expression or activity of a FLJ39501 gene or polypeptide for the manufacture of a medicament (or pharmaceutical composition) for the treatment of a skin disorder.

The invention also resides in a method of treating a skin disorder in a mammalian subject, comprising administering to the subject an effective amount of a compound that modulates the expression or activity of a FLJ39501 gene or polypeptide.

The compound preferably stimulates or mimics the activity of a FLJ39501 protein. The compound may be identified using a method as described above. The compound may be a FLJ39501 protein or a corresponding nucleic acid.

A further object of this invention resides in the use of 20-carboxy-(R)-trioxilin A3 or an analog thereof, as well as any pharmaceutically acceptable salt or hydrate thereof, for the manufacture of a pharmaceutical composition for the treatment of a skin disorder, particularly for skin hydration.

A further aspect of the invention also resides in a method of treating a skin disorder in a mammalian subject, comprising administering to the subject an effective amount of 20-carboxy-(R)-trioxilin A3 or an analog thereof, as well as any pharmaceutically acceptable salt or hydrate thereof.

A further object of this invention resides in the use of 20-carboxy-(S)-trioxilin A3, or an analog thereof, as well as any pharmaceutically acceptable salt or hydrate thereof, for the manufacture of a pharmaceutical composition for the treatment of spastic paraplegia and for the maintenance of neuronal integrity, as well as in a corresponding method of treatment.

Another object of this invention is a composition, particularly a pharmaceutical or cosmetic composition, comprising 20-carboxy-(R)-trioxilin A3 or an analog thereof, including any pharmaceutically acceptable salt or hydrate thereof, and a pharmaceutically acceptable carrier, preferably for topical application.

Still another object of this invention is 20-carboxy-(R)-trioxilin A3 or an analog thereof, including any pharmaceutically acceptable salt or hydrate thereof, as a medicament or a cosmetic product, particularly for the treatment of dry skin.

These compounds may be prepared in vitro from arachidonic acid using native or recombinant enzymes, or through synthetic methods, using standard chemical procedures.

An analog of the compound may be any compound having the same type of biological activity. Typically, the analog is a synthetic molecule, having essentially the same core structure, which may contain additional or alternative substituting groups or chemical functions. Such analogs may be produced by drug design techniques, which are known in the art. Analogs include substituted or derivatized forms of the metabolite having an increased stability, in vivo half-life or which can be more easily produced. The term analog particularly encompasses a pro-medicament, i.e., a compound that is transformed in vivo into the metabolite. The term analog also includes agonist compounds, i.e., compounds that bind the same receptor as the above-referenced molecules and mediate essentially the same biological effect. Analogs may be screened using any one of the above disclosed screening methods.

The compounds may be administered through various routes, including oral, systemic and topical routes, particularly by topical administration. The compounds may be formulated in any appropriate buffer or excipient, such as saline solutions, isotonic buffer, gel, paste, ointment, etc.

For topical administration, the composition may be in the form of an ointment, gel, cream, soap, foam, salve, spray and the like. Suitable excipients include any vehicle or agent that is non-toxic in vivo. For topical application to the skin, the composition may be in the form of an aqueous or oily solution, suspension or dispersion, which may be liquid or semi-liquid, such as a lotion, serum, milky preparation, etc. The composition may be in emulsified form, either oil-in-water or water-in-oil can. It may also be in the form of an anhydrous cream or gel, microparticles, dispersion, etc.

Suitable excipients may include solubilizing agents, stabilizing agents, penetrants, emulsifying agents, surfactants, etc., either alone or in combination(s). The cosmetic composition may also contain additional agents such as gelling agents, antioxidants, solvents, flavoring agents, preservatives, etc. The respective amounts of these agents may be adjusted by the skilled artisan, typically within the range of 0.01% to 15% of the total weight of the composition. Suitable emulsifiers for use in the present invention include, without limitation, glycerol, polysorbate and stearate. Suitable gelling agents include, without limitation, carboxyvinyl and acrylic copolymers, polysaccharides (e.g., hydroxypropylcellulose), polyacrylamides, clays, aluminium stearates, ethylcellulose and polyethylene. If appropriate, for topical administration, a thickening agent such as methyl cellulose may be used as well.

For oral administration, the compounds may be formulated into any conventional dosage form, including tablets, capsules, ampoules, etc. For transmucosal or transdermic administration, they may be formulated in the presence of penetrants, as gels, sprays, patch, etc.

The above methods, uses and compositions can be used to treat skin disorders, either alone or in combination with other agents, including to reduce the progression, prevent the development, suppress any symptoms or completely abolish the disease. The compounds may be administered according to various protocols, which may be adjusted by the practitioner, including the use of repeated administrations. Typical dosages may vary for instance from 0,01 to 1000 mg of active agent per dose.

As disclosed above, the skin disorder is selected from a group of dry skin disorders, particularly a dermatosis such as atopic skin, contact dermatitis, eczema, a psoriasis, or a keratinization disorder such as an ichthyosis and a palmoplantar keratoderma.

In another aspect, the skin disorder is selected from any skin inflammation or aggression, including burnt skin, acid, UV, etc.

The invention also relates, generally, to a pharmaceutical composition comprising a FLJ39501 protein, or an agonist or an antagonist thereof, in combination with a pharmaceutically acceptable vehicle or excipient, particularly for topical administration.

Furthermore, 20-carboxy-(S)-trioxilin A3 and 20-carboxy-(R)-trioxilin A3 may also be used to isolate their corresponding receptor, as disclosed above. In this regard, a particular object of this invention also resides in a compound selected from 20-carboxy-(S)-trioxilin A3 and 20-carboxy-(R)-trioxilin A3, wherein said compound comprises a detectable label. The label may be e.g., any chemical, biological or physical label, such as a luminescent moiety, a radioactive moiety, a fluorescent dye, an enzyme, etc.

Further aspects and advantages of this invention will be disclosed in the following examples, which should be considered as illustrative and not limiting the scope of this application.

### LEGENDS TO THE FIGURES

Figure 1: Proteins of known and inferred lipoxygenase pathways. The 5-lipoxygenase (leukotriene) pathway which has been studied extensively is presented as a model. The 12-lipoxygenase (hepoxilin) pathway with R-chirality is shown; the parallel pathway with S-chirality (not shown) is analogous. On the left, the classes of proteins (enzyme, transporter, receptor), known to be implicated at each step are listed. The proteins, when identified, are represented directly on the figure between an upstream and a downstream product, and are in blue. The hypothetical proteins are in yellow. For simplicity, not all reactions are shown, such as ω-hydroxylation of HXA3.

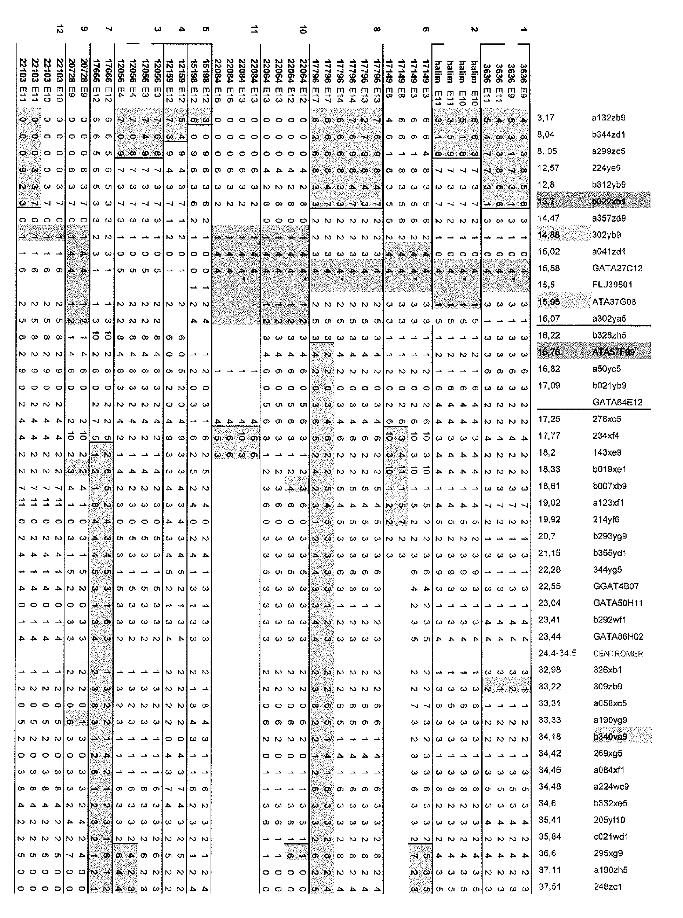

Figure 2: Patients' haplotypes. Loss of homozygosity is indicated by gray color. Inside the smallest segregating interval between markers (pink) b022xb1 and ATA57F09 mutations and common alleles are in green. The allele number is indicated as 0 when no genotyping result is available. Paternal and maternal alleles for each affected child are presented.
Figure 3: Nucleotide and amino acid sequence of a FLJ39501 gene coding portion and polypeptide. The coding region starts with nucleotide 168 and ends up with nucleotide 1763.

### EXAMPLES

### MATERIALS AND METHODS

### Subjects and samples

Three dermatologists (B.B, R.C. and A.M.) recorded the clinical data and pedigree information of the families. Blood samples were collected from each participating family member after obtaining written informed consent. DNA extraction from peripheral blood leukocytes and establishment of cell lines were performed at the DNA bank of Généthon using standard procedures.

### Genetic analysis

Genotyping was carried out using 400 highly polymorphic microsatellite markers from the ABI panel (Linkage Mapping Set2, LMS2, Applied Biosystems) and a MegaBase capillary sequencer for the genome-wide scan. ABI 377 sequencers were used for fine mapping with publicly available microsatellites. Haplotypes were constructed assuming the most parsimonious linkage phase. Linkage programs were used based on the assumption of autosomal recessive inheritance, full penetrance and a disease frequency of 1/300,000 in the general population. Pairwise LOD scores were calculated with the MLINK program of the LINKAGE 5.1 package (54) incorporating consanguineous loops into the pedigree files. For linkage-disequilibrium analysis, the excess of the disease-associated alleles was calculated as previously described (13) by the P_{excess} equation: P_{excess} = (P_{affected} -Pₙₒᵣₘₐₗ)/(1-Pₙₒᵣₘₐₗ), in which P_{affected} and Pₙₒᵣₘₐₗ denote respectively, the frequency of the disease-associated allele on 11 disease-bearing chromosomes and 20 normal chromosomes (55). Chromosomes that either were shared by two sibs or were homozygous were counted only once. For χ² estimation, we used the combined-allele method (56), corrected by Bonferroni's procedure (57).

### Mutation screening

Mutation analysis was performed in the 12 families in affected patients, in both parents, and in supplementary non-affected sibs. We designed intronic oligonucleotide primers flanking the exons for amplification and sequencing of the *FLJ39501* gene (Table 2) using the Primer3 program (http://www-genome.wi.mit.edu/genome_sofhtware/other/primer3.html) (58). Sets of PCR conditions were used as indicated in Table 2. The touch-down PCR reaction was performed in a 45 µl volume containing 50 ng of genomic DNA (in 5 µl) with Hot Master^{™} Taq DNA polymerase (Eppendorf): initial denaturation step at 95 °C for 5 min, 6 cycles of amplification consisting of 40 s at 94 °C, 30 s 68 °C, and a 30 s elongation step at 72 °C, followed by 30 cycles of 40 s at 94 °C, 30 s at optimal annealing temperature, 30 s at 72 °C, and a 5 min terminal elongation step. One to 2 µl of purified PCR products were added to 0.5 µl of sense or antisense primer (20 µM) and 2 µl of BigDye terminator mix (Applied Biosystems) in a 15 µl volume. The linear amplification consisted of an initial 5 min denaturation step at 96 °C, 25 cycles of 10 s of denaturation at 96 °C and a 4 min annealing/extension step at 56-60 °C. The reaction products were purified and sequenced on an Applied Biosystems Sequencer 3700. The forward or reverse strands from all patients and controls were sequenced for the entire coding region and the exon/intron boundaries. The sequences were analysed with the Phred Phrap program on Unix.

### Lymphoblastoid, keratinocyte and fibroblast cell cultures, and RNA extraction

Lymphoblastoid cell lines were established using standard procedures. Total RNA from lymphocytes was extracted with the RNA-PLUS (Quantum-Appligene) kit following the manufacturer's instructions. Human keratinocytes and fibroblasts were obtained from skin removed during routine plastic surgery of a normal individual. The skin sample was processed for primary keratinocyte culture and cells were grown according to the standard procedure described by Invitrogen Life Technologies using products from the same company in serum-free keratinocyte medium supplemented with bovine pituitary extract (25 microg/ml) and recombinant epidermal growth factor (0.1 ng/ml). For primary fibroblast culture we used DMEM (Dulbecco's Modified Eagle's Medium) with 10% fetal calf serum and 2% L-glutamine. Cultures were grown for two passages and harvested when they reached 90% confluence. Total RNA was isolated using the QIAamp RNA Mini Protocol for isolation of total RNA from cultured cells (QIAGEN) following the manufacturer's instructions. The mRNA was isolated following the Oligotex direct mRNA protocol as provided by the manufacturer (QIAGEN).

### RT-PCR and EXPRESSION

RT-PCR was performed using the RT-PCR kit (Invitrogen) with oligo dT primers to generate the first strand of cDNA. Amplification of cDNA from cultured keratinocytes, fibroblasts, placenta and lymphocytes, bone marrow, small intestine, bladder, liver, skeletal muscle, testis, brain and kidney was performed with 2 primer pairs (Table 2) covering the entire coding region, the 3'UTR and the 5'UTR region.

### Additional Accession numbers

ABHD5, (NP_057090); ALDH3A2, (NP_000373); CYP4A11, (NP_001073); CYP2B6, (NP_000761); CYP2B19, (NP_031840); CYP4F2, (NP_001073); CYP4F3, (NP_000887); Ichthyin, (XP_351633); LTB4R1, (NP_858043); LTB4R2, (NP_062813); NIPA1, (NP_653200).

### Online Mendelian Inheritance in Man (http://www.ncbi.nlm.nih.gov/Omim)

Abhydrolase domain containing 5 (*ABHD5*), previously *CGI58,* Comparative Gene Identification 58 [*CGI58*; MIM 604780]; adrenal congenital hyperplasia, [MIM,118485, 201710, 201910 and 202110]; Antley-Bixler syndrome [MIM 207410]; Arachidonate lipoxygenase 3 [*ALOXE3*; MIM 607206]; Arachidonate 12-lipoxygenase, R type [*ALOX12B;* MIM 603741]; ATP-binding cassette, subfamily A, member 12 [*ABCA12*; MIM 607800]; Bile acid synthesis defects [MIM 118455 and 603711]; Cerebrotendinous xanthomatosis [CTX; MIM 213700]; Chanarin-Dorfinan syndrome [CDS; MIM 275630]; Congenital hypoaldosteronism [CMOI; MIM 203400]; Ichthyosis X-linked [STS; MIM 607800]; Ichthyosis nonlamellar and nonerythrodermic congenital [NNCI; MIM 604781]; Lamellar ichthyosis [LI; MIM 242300]; Lamellar ichthyosis 1 [LI1; MIM 604777]; Lamellar ichthyosis 2 [LI2; MIM 601277]; Lamellar ichthyosis 3 [LI3; MIM 604777]; Lamellar ichthyosis 5 [LI5; MIM 606545]; Leukotriene B4 receptor [*LTB4R*; MIM 601531]; Leukotriene B4 receptor 2 [*LTB4R2*; MIM 605773]; Nonbullous ichthyosiform erythroderma [NCIE1, MIM 242100]; Nonbullous ichthyosiform erythroderma [NCIE2, MIM 604780]; Non-imprinted gene in Prader-Willi syndrome/Angelman syndrome chromosome region 1 [*NIPA1*; MIM 6008145]; Primary infantile glaucoma [GLC3A, MIM 231300]; STS; Sjögren-Larsson syndrome [SLS, ALDH3A2, MIM 270200]; Transglutaminase 1 [*TGM1*; MIM 190195]; Spastic paraplegia 6, autosomal dominant [SPG6; MIM 600363].

### RESULTS

### Clinical description, histological features and patient origins

We analyzed 12 consanguineous (first cousin marriages) families comprising 21 patients (12 females, 9 males) and 49 non-affected family members. All families were from Mediterranean countries: nine from Algeria, one from France, one from Italy, and one from Lebanon. Most of the patients were not born as collodion babies but presented a more erythrodermal status of the skin at birth. After the birth, they presented clinical aspects of generalized lamellar ichthyosis with whitish, grayish scaling which was more exaggerated in the peri-umbilical region, on the lower part of the body, and on the buttocks. Hyperlinearity of palms and soles was observed in all patients similar to that found in ichthyosis vulgaris. Some of them showed a more severe lamellar ichthyosis phenotype with dark brownish scales arranged in parallel. There are scales on the scalp in all patients which sometimes have a squamous pytiriasiform appearance.

Light microscopy of skin biopsies revealed typical histopathological features of ichthyosis including hyperkeratosis or more extensive orthohyperkeratosis, mild thickening of the stratum corneum and moderate acanthosis and parakeratosis. A normal or lightly prominent granular layer and a mild dermal perivascular lymphocytic infiltrate with dilatation of dermal capillaries were observed.

### Linkage, linkage disequilibrium and haplotype analysis

A total of 45 microsatellites were genotyped on chromosome 19p12-q12 between the markers D19S424 (AFMal32zb9) and D19S225 (AFM248zcl) to define the smallest common interval and to find an eventual linkage disequilibrium. The maximum pairwise LOD score at θ = 0.00 for D19S930 (AFMa050yc5) was 15.83. A co-segregating region of 3.06 Mb was homozygous in all patients: it was defined by recombination events with loss of homozygosity in six patients from 3 families (F1, F8 and F12) for the telomeric marker AFMb022xb1 (D19S840), and in a patient from family 8 for the centromeric marker ATA57F09 (D19S1171). The results of genotyping are presented in Figure 2 where the haplotypes of patients showing recombinations are shaded in gray. Patients from seven families (F1, F2, F6, F8-F11) shared a common allele for the marker GATA27C12 for which linkage disequilibrium analysis showed a P_{excss} value of 0.64 (χ²: p>0.05 ). In patients from three of these seven families, a larger common haplotype was preserved (1-4-4-1-2). Six of these seven families carried the same mutation. These six families were all from Algeria with the exception of one family (F1) which had French origins.

### Exclusion of candidate genes and identification of mutations in a novel gene

More than 90 known genes or cDNA were located in the initial interval of 3.06 Mb between the markers AFMb022xbl and ATA57F09 (GoldenPath). The known genes which we considered as the best candidate genes, either because of their expression in epidermal tissue, or because of domains and functions which could be implicated in lipid metabolism and particularly the 12-lipoxygenase pathways, were sequenced first. No mutations were found in the coding regions or exon-intron boundaries of 28 of these genes, including a cluster of five cytochromes P450 (CYP) subfamily F genes (cen-CYP4F11, CYP4F2, CYP4F12, CYP4F3, CYP4F8-tel). A new gene, FLJ39501, which was situated next to this cluster in the telomeric direction was of particular interest because it showed homology to CYP4F2 and was expressed in epidermal tissues, including skin and keratinocytes. Sequencing of this gene revealed seven different mutations in patients with ARCI linked to chromosome 19.

### Genomic structure of the FLJ39501 gene and its cDNA

The cDNA of FLJ39501 (AK096820) is 2608 bp long, and is identified as full-length (31). It codes for a protein of 531 amino acids (BAC04868). Public databases (Ensembl, NCBI) supported the existence of 12 exons. The sequence was checked by overlapping RT-PCR, and the products were sequenced and compared with the sequences from public databases. BLAST analysis revealed strong homologies with mouse (NM_177307) and rat mRNA orthologs (XM_234837.1) showing homology of 94 % and 86 % for the nucleotide sequence, and of 64.80 % and 63.25 % for the protein sequence respectively. Multiple nucleotide alignments (http://prodes.toulouse.inra.fr/multalin/) of orthologs from human, mouse and rat also showed a highly conserved coding sequence with homologies of around 80% over a length of about 1800 bp. This gene is also highly conserved in other eukaryotes, such as *Arabidopsis thaliana* and *Orysa sativa.*

### Mutation analysis of the FLJ39501 gene

Sequencing of the 12 exons and of the exon-intron boundaries of the gene revealed seven different homozygous mutations in the 12 consanguineous families (Table 1): one was a large deletion in which exons 3-12 were missing (F3), another one was a small deletion of one bp (980de1C) in exon 7 (F9) which leads to a frameshift and a stop codon, and five were missense mutations: 177C→G (F59L) in exon 1 (F5); 728G→A (R243H) in exon 6 (F4); 1114C→T (R372W) in exon 8 (F7); and 1303C→T (H435Y) (F1, F2, F6, F8, F10, and F11), and 1306C→G (H436D) in exon 10 (F12). The same 1303C→T (H435Y) mutation was shared by six families, all of which were from Algeria with the exception of family F1 which had French origins. The missense mutations were all situated in parts of the gene that are highly conserved between mice, rats and humans. None of these sequence variations were found in 100 normal chromosomes from a Mediterranean control population.

### Expression analysis

We analyzed tissue expression by *FLJ39501*-specific RT-PCR with primer pairs RT_1 (Table 2) for a 412 bp fragment using RNAs from cultured keratinocytes, lymphocytes, fibroblasts, placenta, bone marrow, small intestine, bladder, liver, skeletal muscle, testis, brain and kidney. The *FLJ39501* transcript was found to be expressed in half of the tissues tested; expression was high in cultured keratinocytes, lower in kidney, testis and brain, and very low in placenta and bone marrow. No expression was detectable the other tissues tested.

### Sequence analysis of the FLJ39501 protein and identification of conserved residues

The sequence of 531 amino acids (aa) corresponds to a protein with a calculated molecular weight of 61.96 kDa. A signal peptide was predicted by several programs such as SignalP, with cleavage between aa 48 and 49. According to databases of protein family domains, a CYP motif spans aa 60 to 524 (PFAM, PF00067), and a CYP cysteine heme-iron ligand was found by PROSITE between aa 468 and 477 (PS00086). Several protein fingerprints, which characterize CYP, are also identified by PRINTS: PR00385: CYP, aa 335-352, 390-401, 466,475, 486; PR00463: EP450I, aa 324-341, 344-370, 430-454, 465-475, 475-498; PR00464: EP450II, aa 149-169, 205-223, 424-439; PR00465: EP450IV, aa 326-352, 385-401,435-453,459-475,475-493.

FLJ39501 encodes a protein which was found to be a CYP, family 2, subfamily E, polypeptide 2 homolog of the leukotriene B4-ω-hydroxylase (CYP4F2), which is also known as leukotriene-B4-20-monooxygenase. Homology between these two proteins over 505 aa is 66.21%.

### DISCUSSION

The identification of mutations in FLJ39501 through genetic analysis provides an opportunity to study the role of a CYP gene, and its mechanism of action in a metabolic context. With the exception of polymorphisms in several CYP genes described in a wide variety of disorders, deleterious mutations in human CYP have been found in only a few genetic diseases: several steroidogenesis disorders (Antley-Bixler syndrome, MIM 207410; adrenal congenital hyperplasia, MIM 118485, 201910, 201750, and 202110; cerebrotendinous xanthomatosis, MIM 213700; congenital hypoaldosteronism, MIM 203400), primary infantile glaucoma (MIM 231300), and two different isolated cases with bile acid synthesis defects (MIM 118455 and 603711).

The CYP proteins constitute a superfamily of heme-thiolate proteins. CYP enzymes usually act either as terminal oxidases in multicomponent electron transfer chains, or as monooxygenase systems involved in the metabolism of a plethora of both exogenous and endogenous compounds, including those of arachidonic acid metabolism and eicosanoid biosynthesis (33). Fifty-seven human CYP genes have been described to date and have been categorized into 9 clans, 18 families and 43 subfamilies by their sequence similarities (http://drnelson.utmem.edu/P450lect.html) (33). The physiological reactions catalyzed by CYP enzymes and their substrates have been difficult to elucidate because of the large number of genes and splicing variants (with different affinities for the same substrate) (34), their catalytic versatility and consequently the high number of potential substrates, their variability of expression in the tissues or organisms analyzed, and the difference between *in vitro* versus *in vivo* studies (35,36). In eicosanoid metabolism, CYP enzymes have been subdivided into three groups: 1) the epoxygenases, which catalyse the synthesis of epoxyeicosatrienoic acids (EET), primarily the CYP2C and 2J isoforms in humans, 2) the ω-oxidases, primarily the CYP4A and 4F isoforms in humans, and 3) the allylic oxidases which synthesize several midchain conjugated dienols, primarily CYP4A in humans (35,36). FLJ39501 has been annotated, by sequence homology, as a CYP, family 2, subfamily E, polypeptide 2 homolog of the leukotriene B4-ω-hydroxylase. CYP4F2 was found to be the main LTB4-ω-hydroxylating enzyme in human liver and kidney (37), but not in polymorphonuclear leukocytes where it is not expressed, and where the reaction has been found to be catalysed by CYP4F3, another close homolog of CYP4F2 (87.3 % aa similarity) (38,39).

The phenotype caused by mutations in FLJ39501 is similar to that previously described for other ARCI, in which the majority of patients present a NCIE phenotype at birth, progressing later to a more lamellar aspect of the skin (8-14). Hyperlinearity of palms and soles, and an unusual type of palmoplantar keratoderma seem to characterize this form of ARCI.

As was the case for the 6 genes previously identified in ARCI and in Chanarin-Dorfinan syndrome (MIM 275630) (8-13), homozygosity mapping proved to be an efficient method for localization of the causative genes. One difficulty in the present study was the refinement of the interval which initially encompassed the centromeric region in which usually only few recombinations are observed. Situated on chromosome 19 which is very rich in genes, the smallest common interval of 3.06 Mb still contains over 90 genes. In 2000, a Finnish group described a novel localization for ARCI on chromosome 19p13.1-p13.2 between the markers D19S221 and D19S885 in a single large kindred (32). Their 3.5 Mb interval (12,57 Mb -16,07 Mb) is nearly identical to our refined 3.06 interval (13,7-16,76 Mb), but the Finnish phenotype was described as a mild nonerythrodermic, nonlamellar ichthyosis which did not resemble classical LI or NCIE. As in our patients, palmoplantar hyperlinearity was present; this clinical characteristic is uncommonl in other forms of ARCI.

The mutations found in *FLJ39501* include a large deletion which excises 10 of the 12 exons, and another small deletion in exon 7 which would lead to the synthesis of a truncated protein. All the missense mutations, except F59L, are situated inside the large CYP domain as defined by the PFAM database (PF00067). Furthermore the two neighboring mutations, H435Y and H436D, were also situated in the overlapping fingerprint domains EP450I, EP450II, EP450IV, which characterize CYP proteins (PRINTS: PR00463 and PR00464).

The finding of a CYP gene involved in a form of ARCI fits with the suspected enzymes of the 12(R)-lipoxygenase pathway (Figure 1). Following the model of the leukotriene pathway in which ω-hydroxylation was mainly described for LTB4, and with the assumption that CGI58/ABHD5 is an epoxide hydrolase, trioxilin appears to be the physiological substrate of FLJ39501, at least in the skin, and thus represents a valuable therapeutic metabolite.

The present invention thus reopens exciting avenues for the patients in the mechanisms and treatments of skin disorders (particularly skin hydration) and neuronal degeneration.

**Table 1: Origin of families and mutations**

| Family | Number patients | Origin | Mutation | Effect | Exon |
|---|---|---|---|---|---|
| 1 | 2 | France | 1303C→T | H435Y | 10 |
| 2 | 2 | Algeria | 1303C→T | H435Y | 10 |
| 6 | 2 | Algeria | 1303C→T | H435Y | 10 |
| 8 | 3 | Algeria | 1303C→T | H435Y | 10 |
| 10 | 2 | Algeria | 1303C→T | H435Y | 10 |
| 11 | 2 | Algeria | 1303C→T | H435Y | 10 |
| 3 | 2 | Italy | Large deletion | | 3-12 |
| 4 | 1 | Algeria | 728G→A | R243H | 6 |
| 5 | 1 | Algeria | 177C→G | F59L | 1 |
| 7 | 1 | Algeria | 1114C→T | R372W | 8 |
| 9 | 1 | Libanon | 980delC | frameshift | 7 |
| 12 | 2 | Algeria | 1306C→G | H436D | 10 |

### Table 2 : Primer sequences for FLJ39501 : exons amplification and RT_PCR

**Table 2A: Exon amplification for FLJ39501**

| Nar | Forward sequence | Reverse sequence | PCR_ conditions | Length (bp) of amplicon | SEQ ID # |
|---|---|---|---|---|---|
| 1 | gtgtgctgggaaccttctgt | aaactgcttgccctctctga | Hot 60 | 492 | 3,4 |
| 2 | agccaactgcctgaaatcat | tcaaatgacccttcctctgg | Hot 60 | 374 | 5,6 |
| 3 | tggatgacagagcaagactc c | tccacttgtcacctttgctg | Hot 60 | 296 | 7,8 |
| 4 | ggctggggctttagagaaga | agcctaacaaggacccgact | Hot 60 | 382 | 9,10 |
| 5 | ggtccaggctccaactcat | tcccataggccagagttgtc | Hot 60 | 388 | 11,12 |
| 6 | aatggggacaggaggcttat | caccacgcctaatggagttt | Hot 60 | 457 | 13,14 |
| 7 | tgcagttagccgagattgtg | tggatgttgtgtcgtgacct | Hot 60 | 373 | 15,16 |
| 8 | tgtttgagggtgaggatgtg | cccccattttgtagctgaag | Hot 60 | 398 | 17,18 |
| 9 | gtggctcggcctctagttat | cccctgtggacaatagagca | Hot 60 | 242 | 19,20 |
| 10 | atggctcatgggaacatcat | aaatggctaaatgcggagtg | Hot 60 | 298 | 21,22 |
| 11 | tgctccccatccatctttac | tggtgctcaatacccaggat | Hot 60 | 381 | 23,24 |
| 12 | gcgtggggtttcactttaac | ctatgccctcgggatcttt | Hot 60 | 391 | 25,26 |

**Table 2B : RT PCR Expression**

| Name | Forward | Reverse | Conditions | Length | SEQ ID # |
|---|---|---|---|---|---|
| RT_exp 1 | ctggccctaaagcaaggac | acaggtcccatccataccaag | Takara 58 C | 412 | 27,28 |
| RT_exp2 | ctggccctaaagcaaggac | agtcagatcgtcccactcca | Takara 58 C | 1254 | 29,30 |

| | | | | | |
|---|---|---|---|---|---|
| RT_expl 1 = primers for expressio RT_exp2 = amplification of cDNA (not complete, 3' part missing) | | | | | |

### REFERENCES

1. Williams, M.L. and Elias, P.M. (1985) Heterogeneity in autosomal recessive ichthyosis. Clinical and biochemical differentiation of lamellar ichthyosis and nonbullous congenital ichthyosiform erythroderma. Arch. Dermatol., 121, 477-488.
2. Traupe, H. (1989) The ichthyoses: a guide to clinical diagnosis, genetic counseling, and therapy. Springer, Heidelberg, Germany.
3. Griffiths, W.A.D., Judge, M.R. and Leigh, I.M. (1998) Disorders of keratinization. In: Champion, R.H., Burton, J.L., Bums, D.A. and Breathnach, S.M. (eds) Rook/Wilkinson/Ebling: Textbook of Dermatology. Blackwell Science, Oxford, pp 1483-1530.
4. Akiyama, M., Sawamura, D. and Shimizu, H. (2003) The clinical spectrum of nonbullous congenital ichthyosiform erythroderma and lamellar ichthyosis. Clin. Exp. Dermatol., 28, 235-240.
5. Madison, K.C. Barrier function of the skin: "la raison d'être" of the epidermis. J. Invest. Dermatol., 121, 231-241.
6. Lavrijsen, A.P., Bouwstra, J.A., Gooris, G.S., Weerheim, A., Bodde, H.E. and Ponec, M. (1995) Reduced skin barrier function parallels abnormal stratum corneum lipid organization in patients with lamellar ichthyosis. J. Invest. Dermatol., 105, 619-624.
7. Melnick, B. (1989) Epidermal lipids and the biochemistry of keratinization. In: Traupe, H. (ed) The ichthyoses: a guide to clinical diagnosis, genetic counseling, and therapy. Springer, Heidelberg, Germany, pp 14-42.
8. Huber, M., Rettler, I., Bernasconi, K., Frenk, E., Lavrijsen, S.P., Ponec, M., Bon, A., Lautenschlager, S., Schorderet, D.F. and Hohl, D. (1995) Mutations of keratinocyte transglutaminase in lamellar ichthyosis. Science, 267, 525-528.
9. Russell, L.J., DiGiovanna, J.J., Rogers, G.R., Steinert, P.M., Hashem, N., Compton, J.G. and Bale, S.J. (1995) Mutations in the gene for transglutaminase 1 in autosomal recessive lamellar ichthyosis. Nat. Genet., 9, 279-283.
10. Jobard, F., Lefèvre, C., Karaduman, A., Blanchet-Bardon, C., Emre, S., Weissenbach, J., Özgüc, M., Lathrop, M., Prud'homme, J.F. and Fischer, J. (2002) Lipoxygenase-3 (ALOXE3) and 12(R)-lipoxygenase (ALOX12B) are mutated in non-bullous congenital ichthyosiform erythroderma (NCIE) linked to chromosome 17p13.1. Hum. Mol. Genet., 11, 107-113.
11. Lefèvre, C., Audebert, S., Jobard, F., Bouadjar, B., Lakhdar, H., Boughdene-Stambouli, O., Blanchet-Bardon, C., Heilig, R., Foglio, M., Weissenbach J. et al. (2003) Mutations in the transporter ABCA12 are associated with lamellar ichthyosis type 2. Hum. Mol. Genet., 12, 2369-2378.
12. Lefèvre, C. Bouadjar, B., Karaduman, A., Jobard, F., Saker, S., Ozgüc, M., Lathrop, M., Prud'homme, J-F. and Fischer, J. (2004) Mutations in ichthyin a new gene on chromosome 5q33 in a new form of autosomal recessive congenital ichthyosis. Hum. Mol. Genet., 13, 2473-2482.
13. Lefèvre, C., Jobard, F., Caux, F., Bouadjar, B., Karaduman, A., Heilig, R., Lakhdar, H., Wollenberg, A., Verret, J.L., Weissenbach J. et al. (2001) Mutations in CGI-58, the gene encoding a new protein of the esterase/lipase/thioesterase subfamily, in Chanarin-Dorfinan syndrome. Am. J. Hum. Genet., 69, 1002-1012.
14. Richard, G. (2004) Molecular genetics of the ichthyoses. Am. J. Med. Genet., 131C, 32-44.
15. Candi, E., Schmidt, R. and Melino, G. (2005) The cornified envelope: a model of cell death in the skin. Nat. Rev. Mol. Cell. Biol., 4,328-340.
16. Kelsell, D.P., Norgett, E.E., Unsworth, H., Teh, M.T., Cullup, T., Mein CA, Dopping-Hepenstal PJ, Dale BA, Tadini G, Fleckman P, et al. (2005) Mutations in ABCA12 underlie the severe congenital skin disease Harlequin ichthyosis. Am. J. Hum. Genet., 76, 794-803.
17. Elias, P.M., Crumrine, D., Rassner, U., Hachem, J.P., Menon, G.K., Man, W., Choy, M.H, Leypoldt, L., Feingold, K.R. and Williams, M.L. (2004) Basis for abnormal desquamation and permeability barrier dysfunction in RXLI. J. Invest. Dermatol., 122, 314-319.
18. Pace-Asciak, C.R. (1994) Hepoxilins: a review on their cellular actions. Biochim. Biophys. Acta., 1215, 1-8.
19. Pace-Asciak, C.R., Reynaud, D., Demin, P. and Nigam, S. (1999) The hepoxilins. A review. Adv. Exp. Med. Biol., 447, 123-132.
20. Anton, R. and Vila, L. (2000) Stereoselective biosynthesis of hepoxilin B3 in human epidermis. J. Invest. Dermatol., 114, 554-559.
21. Funk, C.D. (2001) Prostaglandins and leukotrienes: advances in eicosanoid biology. Science, 294, 1871-1875.
22. Anton, R., Camacho, M., Puig, L. and Vila, L. (2002) Hepoxilin B3 and its enzymatically formed derivative trioxilin B3 are incorporated into phospholipids in psoriatic lesions. J. Invest. Dermatol., 118, 139-146.
23. Yu, Z., Schneider, C., Boeglin, W.E., Mamett, L.J. and Brash, A.R. (2003) The lipoxygenase gene ALOXE3 implicated in skin differentiation encodes a hydroperoxide isomerase. Proc. Natl. Acad. Sci. USA, 100, 9162-9167.
24. Newman, J.W., Morisseau, C. and Hammock, B.D. (2004) Epoxide hydrolases: their roles and interactions with lipid metabolism. Prog. Lipid Res., 44, 1-51.
25. Yu, Z., Schneider, C., Boeglin, W.E. and Brash, A.R. (2005) Mutations associated with a congenital form of ichthyosis (NCIE) inactivate the epidermal lipoxygenases 12R-LOX and eLOX3. Biochim. Biophys. Acta, 1686, 238-247.
26. Pfister, S.L., Spitzbarth, N., Nithipatikom, K., Falck, J.R. and Campbell, W.B. (2003) Metabolism of 12-hydroperoxyeicosatetraenoic acid to vasodilatory trioxilin C3 by rabbit aorta. Biochim. Biophys. Acta, 1622, 6-13.
27. Fischer, J., Faure, A., Bouadjar, B., Blanchet-Bardon, C., Karaduman, A., Thomas, I., Emre, S., Cure, S., Özgüc, M., Weissenbach, J., et al. (2000) Two new loci for autosomal recessive ichthyosis on chromosomes 3p21 and 19p12-ql2 and evidence for further genetic heterogeneity. Am. J. Hum. Genet., 66, 904-913.
28. De Laurenzi, V., Rogers, G.R., Hamrock, D.J., Marekov, L.N., Steinert, P.M., Compton, J.G., Markova, N. and Rizzo, W.B. (1996) Sjögren-Larsson syndrome is caused by mutations in the fatty aldehyde dehydrogenase gene. Nat. Genet., 12, 52-57.
29. Rizzo, W.B., Dammann, A.L. and Craft, D.A. (1988) Sjögren-Larsson syndrome. Impaired fatty alcohol oxidation in cultured fibroblasts due to deficient fatty alcohol:nicotinamide adenine dinucleotide oxidoreductase activity. J. Clin. Invest., 81, 738-744.
30. Rizzo, W.B. Sjögren-Larsson syndrome: fatty aldehyde dehydrogenase deficiency. (2001) In: Scriver, C.R., Beaudet, A.L., Sly, W.S., Valle, D., Childs, B., Kinzler, K.W., and Vogelstein, B., (eds) The metabolic and molecular bases of inherited disease, 8th ed., McGraw-Hill, New-York, pp 2239-2258.
31. Strausberg, R.L., Feingold, E.A., Grouse, L.H., Derge, J.G., Klausner, R.D., Collins, F.S., Wagner, L., Shenmen, C.M., Schuler, G.D., Altschul, S.F., et al. (2002) Generation and initial analysis of more than 15,000 full-length human and mouse cDNA sequences. Proc. Natl. Acad. Sci. USA., 99,16899-16903.
32. Virolainen, E., Wessman, M., Hovatta, I., Niemi, K.M., Ignatius, J., Kere, J., Peltonen, L. and Palotie, A. (2000) Assignment of a novel locus for autosomal recessive congenital ichthyosis to chromosome 19p13.1-p13.2. Am. J. Hum. Genet., 66, 1132-1137.
33. Nelson, D.R., Zeldin, D.C., Hoffman, S.M., Maltais, L.J., Wain, H.M. and Nebert, D.W. (2004) Comparison of cytochrome P450 (CYP) genes from the mouse and human genomes, including nomenclature recommendations for genes, pseudogenes and alternative-splice variants. Pharmacogenetics, 14, 1-18.
34. Christmas, P., Jones, J.P., Patten, C.J., Rock, D.A., Zheng, Y., Cheng, S.M., Weber, B.M., Carlesso, N., Scadden, D.T., Rettie, A.E. and Soberman, R.J. (2001) Alternative splicing determines the function of CYP4F3 by switching substrate specificity. J. Biol. Chem., 276, 38166-38172.
35. Capdevila, J.H., Falck, J.R. and Harris, R.C. (2000) Cytochrome P450 and arachidonic acid bioactivation. Molecular and functional properties of the arachidonate monooxygenase. J. Lipid Res., 41, 163-181.
36. Capdevila, J.H. and Falck, J.R. (2002) Biochemical and molecular properties of the cytochrome P450 arachidonic acid monooxygenases. Prostaglandins Other Lipid Mediat., 68-69, 325-344.
37. Du, L., Hoffman, S.M. and Keeney, D.S.(2004) Epidermal CYP2 family cytochromes P450. Toxicol Appl Pharmacol.,; 195, 278-287.
38. Jin, R., Koop, D.R., Raucy, J.L. and Lasker, J.M. (1998) Role of human CYP4F2 in hepatic catabolism of the proinflammatory agent leukotriene B4. Arch. Biochem. Biophys., 359, 89-98.
39. Kikuta, Y., Kusunose, E. and Kusunose, M. (2002) Prostaglandin and leukotriene omega-hydroxylases. Prostaglandins Other Lipid Mediat., 68-69, 345-362. Erratum in: (2003) Prostaglandins Other Lipid Mediat., 70, 361.
40. Zeldin, D.C. (2001) Epoxygenase pathways of arachidonic acid metabolism. J. Biol. Chem., 276, 36059-36062.
41. Lasker, J.M., Chen, W.B., Wolf, I., Bloswick, B.P., Wilson, P.D. and Powell, P.K. (2000) Formation of 20-hydroxyeicosatetraenoic acid, a vasoactive and natriuretic eicosanoid, in human kidney. Role of Cyp4F2 and Cyp4A11. J. Biol. Chem., 275, 4118-4126.
42. Roman, R.J. (2002) P-450 metabolites of arachidonic acid in the control of cardiovascular function. Physiol. Rev., 82, 131-185.
43. Reynaud, D., Rounova, O., Demin, P.M., Pivnitsky, K.K. and Pace-Asciak, C.R. (1997) Hepoxilin A3 is oxidized by human neutrophils into its omega-hydroxy metabolite by an activity independent of LTB4 omega-hydroxylase. Biochim. Biophys. Acta, 1348, 287-298.
44. Sumimoto, H. and Minakami, S. (1990) Oxidation of 20-hydroxyleukotriene B4 to 20-carboxyleukotriene B4 by human neutrophil microsomes. Role of aldehyde dehydrogenase and leukotriene B4 omega-hydroxylase (cytochrome P-450LTB omega) in leukotriene B4 omega-oxidation. J. Biol. Chem., 265, 4348-4353.
45. Naccache, P.H., Molski, T.F., Becker, E.L., Borgeat, P., Picard, S., Vallerand, P. and Sha'afi, R.I. (1982) Specificity of the effect of lipoxygenase metabolites of arachidonic acid on calcium homeostasis in neutrophils. Correlation with functional activity. J. Biol. Chem., 257, 8608-8611.
46. Hansson, G., Lindgren, J.A., Dahlen, S.E., Hedqvist, P. and Samuelsson, B. (1981) Identification and biological activity of novel omega-oxidized metabolites of leukotriene B4 from human leukocytes. FEBS Lett., 130, 107-112.
47. Escalante, B., Erlij, D., Falck, J.R. and McGiff, J.C. (1991) Effect of cytochrome P450 arachidonate metabolites on ion transport in rabbit kidney loop of Henle. Science, 251, 799-802.
48. Kaduce, T.L., Fang, X., Harmon, S.D., Oltman, C.L., Dellsperger, K.C., Teesch, L.M., Gopal V.R., Falck, J.R., Campbell, W.B., Weintraub, N.L. and Spector, A.A. (2004) 20-hydroxyeicosatetraenoic acid (20-HETE) metabolism in coronary endothelial cells. J. Biol. Chem., 279, 2648-2656.
49. Jedlitschky, G., Huber, M., Volkl, A., Muller, M., Leier, I., Muller, J., Lehmann, W.D., Fahimi, H.D. and Keppler, D. (1991) Peroxisomal degradation of leukotrienes by beta-oxidation from the omega-end. J. Biol. Chem., 266, 24763-24772.
50. Rainier, S., Chai, J.H., Tokarz, D., Nicholls, R.D. and Fink, J.K. (2003) NIPA1 gene mutations cause autosomal dominant hereditary spastic paraplegia (SPG6). Am. J. Hum. Genet., 73, 967-971.
51. Chen, S., Song, C., Guo, H., Xu. P., Huang, W., Zhou, Y., Sun, J., Li, C.X., Du,. Y., et al. (2005) Distinct novel mutations affecting the same base in the NIPA1 gene cause autosomal dominant hereditary spastic paraplegia in two Chinese families. Hum. Mutat., 25, 135-141.
52. Reed, J.A., Wilkinson, P.A., Patel, H., Simpson, M.A., Chatonnet, A., Robay, D., Patton, M.A., Crosby, A.H. and Warner, T.T. (2005) A novel NIPA1 mutation associated with a pure form of autosomal dominant hereditary spastic paraplegia. Neurogenetics, 6, 79-84.
53. Hiesinger, P.R., Fayyazuddin, A., Mehta, S.Q., Rosenmund, T., Schulze, K.L., Zhai, R.G., Verstreken, P., Cao, Y., Zhou, Y., Kunz, J. and Bellen, H.J. (2005) The v-ATPase V0 subunit al is required for a late step in synaptic vesicle exocytosis in Drosophila. Cell, 121, 607-620.
54. Lathrop, G.M. and Lalouel, J-M. (1984) Easy calculations of lod scores and genetic risks on small computers. Am. J. Hum. Genet., 36, 460-465.
55. Hästbacka, J., de la Chapelle, A., Kaitila, I., Sistonen, P., Weaver, A. and Lander, E. (1992) Linkage disequilibrium mapping in isolated founder populations: diastrophic dysplasia in Finland. Nat. Genet., 3, 204-211. Erratum in: Nat. Genet., 4, 343.
56. Aksentijevich, I., Pras, E., Gruberg, L., Shen, Y., Holman, K., Helling, S., Prosen, L., Sutherland, G.R., Richards, R.I., Dean, M., et al. (1993) Familial Mediterranean fever (FMF) in Moroccan Jews: demonstration of a founder effect by extended haplotype analysis. Am. J. Hum. Genet., 53, 644-651.
57. Weir BS. 1990. Genetic data analysis. Sinauer, Sunderland, MA.
58. Rozen, S. and Skaletsky, H.J. (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386.

## Claims

1. The use of 20-carboxy-(S)-trioxilin A3, an analog thereof, or a pharmaceutically acceptable salt or hydrate thereof, for the manufacture of a medicament for the treatment of a neurologic disorder.

2. The use of claim 1, wherein the neurologic disorder is selected from a degenerative disorder, particularly spastic paraplegia, multiple sclerosis and amyotrophic lateral sclerosis.

3. A composition comprising 20-carboxy-(S)-trioxilin A3, an analog thereof, or a pharmaceutically acceptable salt or hydrate thereof, and a pharmaceutically acceptable carrier.

4. The use of 20-carboxy-(S)-trioxilin A3 to isolate its corresponding receptor *in vitro.*


SEQUENCE LISTING

<110>  Centre National de Génotypage

<120>  Compositions and Methods for treating skin disorders

<130>  B418

<160>  30

<170>  PatentIn version 3.3

<210>  1
<211>  2608
<212>  DNA
<213>  Homo sapiens


<220>
<221>  CDS
<222>  (168)..(1763)

<400>  1
tgatcccagg tagtggcgcc ggtggatccg gatcgagggc aggaggctga gacccgcggg      60

agctggccct aaagcaagga cctgagtgca agtaattttt ttgggaagta ataacagaaa     120

ataccagcaa ggaagaagac agtgaaccca aaagaattga aaacagg atg ctg ccc      176
                                                    Met Leu Pro
                                                    1

atc aca gac cgc ctg ctg cac ctc ctg ggg ctg gag aag acg gcg ttc      224
Ile Thr Asp Arg Leu Leu His Leu Leu Gly Leu Glu Lys Thr Ala Phe
    5                   10                  15

cgc ata tac gcg gtg tcc acc ctt ctc ctc ttc ctg ctc ttc ttc ctg      272
Arg Ile Tyr Ala Val Ser Thr Leu Leu Leu Phe Leu Leu Phe Phe Leu
20                  25                  30                  35

ttc cgc ctg ctg ctg cgg ttc ctg agg ctc tgc agg agc ttc tac atc      320
Phe Arg Leu Leu Leu Arg Phe Leu Arg Leu Cys Arg Ser Phe Tyr Ile
                40                  45                  50

acc tgc cgc cgg ctg cgc tgc ttc ccc cag cct ccc cgg cgc aac tgg      368
Thr Cys Arg Arg Leu Arg Cys Phe Pro Gln Pro Pro Arg Arg Asn Trp
            55                  60                  65

ctg ctg ggc cac ctg ggc atg tac ctt cca aat gag gcg ggc ctt caa      416
Leu Leu Gly His Leu Gly Met Tyr Leu Pro Asn Glu Ala Gly Leu Gln
        70                  75                  80

gat gag aag aag gta ctg gac aac atg cac cat gta ctc ttg gta tgg      464
Asp Glu Lys Lys Val Leu Asp Asn Met His His Val Leu Leu Val Trp
    85                  90                  95

atg gga cct gtc ctg ccg ctg ttg gtt ctg gtg cac cct gat tac atc      512
Met Gly Pro Val Leu Pro Leu Leu Val Leu Val His Pro Asp Tyr Ile
100                 105                 110                 115

aaa ccc ctt ttg gga gcc tca gct gcc acc gcc ccc aag gat gac ctc      560
Lys Pro Leu Leu Gly Ala Ser Ala Ala Thr Ala Pro Lys Asp Asp Leu
                120                 125                 130

ttc tat ggc ttc cta aaa cct tgg cta ggg gat ggg ctg ctg ctc agc      608
Phe Tyr Gly Phe Leu Lys Pro Trp Leu Gly Asp Gly Leu Leu Leu Ser
            135                 140                 145

aaa ggt gac aag tgg agc cgg cac cgt cgc ctg ctg aca ccc gcc ttc      656
Lys Gly Asp Lys Trp Ser Arg His Arg Arg Leu Leu Thr Pro Ala Phe

150                 155                 160

cac ttt gac atc ctg aag cct tac atg aag atc ttc aac cag agc gct      704
His Phe Asp Ile Leu Lys Pro Tyr Met Lys Ile Phe Asn Gln Ser Ala
    165                 170                 175

gac att atg cat gct aaa tgg cgg cat ctg gca gag ggc tca gcg gtc      752
Asp Ile Met His Ala Lys Trp Arg His Leu Ala Glu Gly Ser Ala Val
180                 185                 190                 195

tcc ctt gat atg ttt gag cat atc agc ctc atg acc ctg gac agt ctt      800
Ser Leu Asp Met Phe Glu His Ile Ser Leu Met Thr Leu Asp Ser Leu
                200                 205                 210

cag aaa tgt gtc ttc agc tac aac agc aac tgc caa gag aag atg agt      848
Gln Lys Cys Val Phe Ser Tyr Asn Ser Asn Cys Gln Glu Lys Met Ser
            215                 220                 225

gat tat atc tcc gct atc att gaa ctg agc gct ctg tct gtc cgg cgc      896
Asp Tyr Ile Ser Ala Ile Ile Glu Leu Ser Ala Leu Ser Val Arg Arg
        230                 235                 240

cag tat cgc ttg cac cac tac ctc gac ttc att tac tac cgc tcg gcg      944
Gln Tyr Arg Leu His His Tyr Leu Asp Phe Ile Tyr Tyr Arg Ser Ala
    245                 250                 255

gat ggg cgg agg ttc cgg cag gcc tgt gac atg gtg cac cac ttc acc      992
Asp Gly Arg Arg Phe Arg Gln Ala Cys Asp Met Val His His Phe Thr
260                 265                 270                 275

act gaa gtc atc cag gaa cgg cgg cgg gca ctg cgt cgg cag ggg gcc     1040
Thr Glu Val Ile Gln Glu Arg Arg Arg Ala Leu Arg Arg Gln Gly Ala
                280                 285                 290

gag gcc tgg ctt aag gcc aag cag ggg aag acc ttg gac ttt att gat     1088
Glu Ala Trp Leu Lys Ala Lys Gln Gly Lys Thr Leu Asp Phe Ile Asp
            295                 300                 305

gtg ctg ctc ctg gcc agg gat gaa gat gga aag gaa ctg tca gac gag     1136
Val Leu Leu Leu Ala Arg Asp Glu Asp Gly Lys Glu Leu Ser Asp Glu
        310                 315                 320

gat atc cga gcc gaa gca gac acc ttc atg ttt gag ggt cac gac aca     1184
Asp Ile Arg Ala Glu Ala Asp Thr Phe Met Phe Glu Gly His Asp Thr
    325                 330                 335

aca tcc agt ggg atc tct tgg atg ctg ttc aat ttg gca aag tat ccg     1232
Thr Ser Ser Gly Ile Ser Trp Met Leu Phe Asn Leu Ala Lys Tyr Pro
340                 345                 350                 355

gaa tac cag gag aaa tgc cga gaa gag att cag gaa gtc atg aaa ggc     1280
Glu Tyr Gln Glu Lys Cys Arg Glu Glu Ile Gln Glu Val Met Lys Gly
                360                 365                 370

cgg gag ctg gag gag ctg gag tgg gac gat ctg act cag ctg ccc ttt     1328
Arg Glu Leu Glu Glu Leu Glu Trp Asp Asp Leu Thr Gln Leu Pro Phe
            375                 380                 385

aca act atg tgc att aag gag agc ctg cgc cag tac cca cct gtc act     1376
Thr Thr Met Cys Ile Lys Glu Ser Leu Arg Gln Tyr Pro Pro Val Thr
        390                 395                 400

ctt gtc tct cgc caa tgc acg gag gac atc aag ctc cca gat ggg cgc     1424
Leu Val Ser Arg Gln Cys Thr Glu Asp Ile Lys Leu Pro Asp Gly Arg
    405                 410                 415

atc atc ccc aaa gga atc atc tgc ttg gtc agc atc tat gga acc cac     1472
Ile Ile Pro Lys Gly Ile Ile Cys Leu Val Ser Ile Tyr Gly Thr His
420                 425                 430                 435

cac aac ccc aca gtg tgg cct gac tcc aag gtg tac aac ccc tac cgc      1520
His Asn Pro Thr Val Trp Pro Asp Ser Lys Val Tyr Asn Pro Tyr Arg
                440                 445                 450

ttt gac ccg gac aac cca cag cag cgc tct cca ctg gcc tat gtg ccc      1568
Phe Asp Pro Asp Asn Pro Gln Gln Arg Ser Pro Leu Ala Tyr Val Pro
            455                 460                 465

ttc tct gca gga ccc agg aat tgc atc gga cag agc ttc gcc atg gcc      1616
Phe Ser Ala Gly Pro Arg Asn Cys Ile Gly Gln Ser Phe Ala Met Ala
        470                 475                 480

gag ttg cgc gtg gtt gtg gca cta aca ctg cta cgt ttc cgc ctg agc      1664
Glu Leu Arg Val Val Val Ala Leu Thr Leu Leu Arg Phe Arg Leu Ser
    485                 490                 495

gtg gac cga acg cgc aag gtg cgg cgg aag ccg gag ctc ata ctg cgc      1712
Val Asp Arg Thr Arg Lys Val Arg Arg Lys Pro Glu Leu Ile Leu Arg
500                 505                 510                 515

acg gag aac ggg ctc tgg ctc aag gtg gag ccg ctg cct ccg cgg gcc      1760
Thr Glu Asn Gly Leu Trp Leu Lys Val Glu Pro Leu Pro Pro Arg Ala
                520                 525                 530

tga gcgtgggcgc gcccctgcgg ctcccgaggg tccaggcccc gcccccaaag           1813

gaccaggact cgccccaaag atcccgaggg cataggccac ccccctcgaa gttcaggttc    1873

agctcctgga tgaccaggca ccgctgttga gcagcctggt ggtactggcc acgcccctca    1933

aggcaaggct ccgcccctta gggggcctga tcccgcccct tgaggcttag gtcccgcccc    1993

ctgacttctc gcacctgtcc tgtttgaggg aacagggtcg accctgcccc cttgggctca    2053

ggccccgccc ccaggatcct acggattgag gtcctcaggc cacgcccctg cagatccagg    2113

tctccaggcc ttgcccactg agccttcaga ggacctaaga cccacctatg actcagggcc     2173

caccacaccc cacccccccc caactggctg aacccctgga aggcttccaa actgaggaaa    2233

gctggagcct gacgtcagag cttttaactc ggacatcacc ctcctgaggc cctcagctca    2293

tacgggcaga ctgctcaggc gtgaggctgg atcgtagggt tcgaggctgc ttgcttttgt    2353

tgttgttttg taaactcaga cccttgtaag ccccttcctt cctcccttgc ccaaagatcc    2413

tttgctgagt gttctgtttt tgcagaataa aagcaaagga tgcagactta ccccaccctc    2473

cacccctagc tcagccagat ccttacacgt ggagccccca gcatgggaat gaggggctct    2533

gcaactgagc gaagctgcgg gctaggaggc tgaaaagcca tttatcctag gacataaact    2593

cagattttgg gggtc                                                     2608

<210>  2
<211>  531
<212>  PRT
<213>  Homo sapiens

<400>  2

Met Leu Pro Ile Thr Asp Arg Leu Leu His Leu Leu Gly Leu Glu Lys
1               5                   10                  15

Thr Ala Phe Arg Ile Tyr Ala Val Ser Thr Leu Leu Leu Phe Leu Leu
            20                  25                  30

Phe Phe Leu Phe Arg Leu Leu Leu Arg Phe Leu Arg Leu Cys Arg Ser
        35                  40                  45

Phe Tyr Ile Thr Cys Arg Arg Leu Arg Cys Phe Pro Gln Pro Pro Arg
    50                  55                  60

Arg Asn Trp Leu Leu Gly His Leu Gly Met Tyr Leu Pro Asn Glu Ala
65                  70                  75                  80

Gly Leu Gln Asp Glu Lys Lys Val Leu Asp Asn Met His His Val Leu
                85                  90                  95

Leu Val Trp Met Gly Pro Val Leu Pro Leu Leu Val Leu Val His Pro
            100                 105                 110

Asp Tyr Ile Lys Pro Leu Leu Gly Ala Ser Ala Ala Thr Ala Pro Lys
        115                 120                 125

Asp Asp Leu Phe Tyr Gly Phe Leu Lys Pro Trp Leu Gly Asp Gly Leu
    130                 135                 140

Leu Leu Ser Lys Gly Asp Lys Trp Ser Arg His Arg Arg Leu Leu Thr
145                 150                 155                 160

Pro Ala Phe His Phe Asp Ile Leu Lys Pro Tyr Met Lys Ile Phe Asn
                165                 170                 175

Gln Ser Ala Asp Ile Met His Ala Lys Trp Arg His Leu Ala Glu Gly
            180                 185                 190

Ser Ala Val Ser Leu Asp Met Phe Glu His Ile Ser Leu Met Thr Leu
        195                 200                 205

Asp Ser Leu Gln Lys Cys Val Phe Ser Tyr Asn Ser Asn Cys Gln Glu
    210                 215                 220

Lys Met Ser Asp Tyr Ile Ser Ala Ile Ile Glu Leu Ser Ala Leu Ser
225                 230                 235                 240

Val Arg Arg Gln Tyr Arg Leu His His Tyr Leu Asp Phe Ile Tyr Tyr
                245                 250                 255

Arg Ser Ala Asp Gly Arg Arg Phe Arg Gln Ala Cys Asp Met Val His
            260                 265                 270

His Phe Thr Thr Glu Val Ile Gln Glu Arg Arg Arg Ala Leu Arg Arg
        275                 280                 285

Gln Gly Ala Glu Ala Trp Leu Lys Ala Lys Gln Gly Lys Thr Leu Asp
    290                 295                 300

Phe Ile Asp Val Leu Leu Leu Ala Arg Asp Glu Asp Gly Lys Glu Leu
305                 310                 315                 320

Ser Asp Glu Asp Ile Arg Ala Glu Ala Asp Thr Phe Met Phe Glu Gly
                325                 330                 335

His Asp Thr Thr Ser Ser Gly Ile Ser Trp Met Leu Phe Asn Leu Ala
            340                 345                 350

Lys Tyr Pro Glu Tyr Gln Glu Lys Cys Arg Glu Glu Ile Gln Glu Val
        355                 360                 365

Met Lys Gly Arg Glu Leu Glu Glu Leu Glu Trp Asp Asp Leu Thr Gln
    370                 375                 380

Leu Pro Phe Thr Thr Met Cys Ile Lys Glu Ser Leu Arg Gln Tyr Pro
385                 390                 395                 400

Pro Val Thr Leu Val Ser Arg Gln Cys Thr Glu Asp Ile Lys Leu Pro
                405                 410                 415

Asp Gly Arg Ile Ile Pro Lys Gly Ile Ile Cys Leu Val Ser Ile Tyr
            420                 425                 430

Gly Thr His His Asn Pro Thr Val Trp Pro Asp Ser Lys Val Tyr Asn
        435                 440                 445

Pro Tyr Arg Phe Asp Pro Asp Asn Pro Gln Gln Arg Ser Pro Leu Ala
    450                 455                 460

Tyr Val Pro Phe Ser Ala Gly Pro Arg Asn Cys Ile Gly Gln Ser Phe
465                 470                 475                 480

Ala Met Ala Glu Leu Arg Val Val Val Ala Leu Thr Leu Leu Arg Phe
                485                 490                 495

Arg Leu Ser Val Asp Arg Thr Arg Lys Val Arg Arg Lys Pro Glu Leu
            500                 505                 510

Ile Leu Arg Thr Glu Asn Gly Leu Trp Leu Lys Val Glu Pro Leu Pro
        515                 520                 525

Pro Arg Ala
    530

<210>  3
<211>  20
<212>  DNA
<213>  Artificial sequence

<220>
<223>  Primer sequences for FLJ39501 exons amplification_ n°1_ Forward
       sequence

<400>  3
gtgtgctggg aaccttctgt                                                   20


<210>  4
<211>  20
<212>  DNA
<213>  Artificial sequence

<220>
<223>  Primer sequences for FLJ39501 exons amplification_ n°1_ Reverse
       sequence

<400>  4
aaactgcttg ccctctctga                                                   20


<210>  5
<211>  20
<212>  DNA
<213>  Artificial sequence

<220>
<223>  Primer sequences for FLJ39501 exons amplification_ n°2_ Forward
       sequence

<400>  5
agccaactgc ctgaaatcat                                                   20


<210>  6
<211>  20
<212>  DNA
<213>  Artificial sequence

<220>
<223>  Primer sequences for FLJ39501 exons amplification_ n°2_ Reverse
       sequence

<400>  6
tcaaatgacc cttcctctgg                                                   20


<210>  7
<211>  21
<212>  DNA
<213>  Artificial sequence

<220>
<223>  Primer sequences for FLJ39501 exons amplification_ n°3_ Forward
       sequence

<400>  7
tggatgacag agcaagactc c                                                 21


<210>  8
<211>  20
<212>  DNA
<213>  Artificial sequence

<220>
<223>  Primer sequences for FLJ39501 exons amplification_ n°3_ Reverse
       sequence

<400>  8
tccacttgtc acctttgctg                                                   20

<210>  9
<211>  20
<212>  DNA
<213>  Artificial sequence

<220>
<223>  Primer sequences for FLJ39501 exons amplification_ n°4_ Forward
       sequence

<400>  9
ggctggggct ttagagaaga                                                   20


<210>  10
<211>  20
<212>  DNA
<213>  Artificial sequence

<220>
<223>  Primer sequences for FLJ39501 exons amplification_ n°4_ Reverse
       sequence

<400>  10
agcctaacaa ggacccgact                                                   20


<210>  11
<211>  19
<212>  DNA
<213>  Artificial sequence

<220>
<223>  Primer sequences for FLJ39501 exons amplification_ n°5_ Forward
       sequence

<400>  11
ggtccaggct ccaactcat                                                    19


<210>  12
<211>  20
<212>  DNA
<213>  Artificial sequence

<220>
<223>  Primer sequences for FLJ39501 exons amplification_ n°5_ Reverse
       sequence

<400>  12
tcccataggc cagagttgtc                                                   20


<210>  13
<211>  20
<212>  DNA
<213>  Artificial sequence

<220>
<223>  Primer sequences for FLJ39501 exons amplification_ n°6_ Forward
       sequence

<400>  13
aatggggaca ggaggcttat                                                   20


<210>  14
<211>  20
<212>  DNA

<213>  Artificial sequence

<220>
<223>  Primer sequences for FLJ39501 exons amplification_ n°6_ Reverse
       sequence

<400>  14
caccacgcct aatggagttt                                                   20


<210>  15
<211>  20
<212>  DNA
<213>  Artificial sequence

<220>
<223>  Primer sequences for FLJ39501 exons amplification_ n°7_ Forward
       sequence

<400>  15
tgcagttagc cgagattgtg                                                   20


<210>  16
<211>  20
<212>  DNA
<213>  Artificial sequence

<220>
<223>  Primer sequences for FLJ39501 exons amplification_ n°7_ Reverse
       sequence

<400>  16
tggatgttgt gtcgtgacct                                                   20


<210>  17
<211>  20
<212>  DNA
<213>  Artificial sequence

<220>
<223>  Primer sequences for FLJ39501 exons amplification_ n°8_ Forward
       sequence

<400>  17
tgtttgaggg tgaggatgtg                                                   20


<210>  18
<211>  20
<212>  DNA
<213>  Artificial sequence

<220>
<223>  Primer sequences for FLJ39501 exons amplification_ n°8_ Reverse
       sequence

<400>  18
cccccatttt gtagctgaag                                                   20


<210>  19
<211>  20
<212>  DNA
<213>  Artificial sequence

<220>
<223>  Primer sequences for FLJ39501 exons amplification_ n°9_ Forward

sequence

<400> 19
gtggctcggc ctctagttat 20

<210> 20
<211> 20
<212> DNA
<213> Artificial sequence

<220>
<223> Primer sequences for FLJ39501 exons amplification_ n°9_ Reverse
sequence

<400> 20
cccctgtgga caatagagca 20

<210> 21
<211> 20
<212> DNA
<213> Artificial sequence

<220>
<223> Primer sequences for FLJ39501 exons amplification_ n°10_ Forward
sequence

<400> 21
atggctcatg ggaacatcat 20

<210> 22
<211> 20
<212> DNA
<213> Artificial sequence

<220>
<223> Primer sequences for FLJ39501 exons amplification_ n°10_ Reverse
sequence

<400> 22
aaatggctaa atgcggagtg 20

<210> 23
<211> 20
<212> DNA
<213> Artificial sequence

<220>
<223> Primer sequences for FLJ39501 exons amplification_ n°11_ Forward
sequence

<400> 23
tgctccccat ccatctttac 20

<210> 24
<211> 20
<212> DNA
<213> Artificial sequence

<220>
<223> Primer sequences for FLJ39501 exons amplification_ n°11_ Reverse
sequence

<400> 24
tggtgctcaa tacccaggat 20

<210>  25
<211>  20
<212>  DNA
<213>  Artificial sequence

<220>
<223>  Primer sequences for FLJ39501 exons amplification_ n°12_ Forward
       sequence

<400>  25
gcgtggggtt tcactttaac                                                   20


<210>  26
<211>  19
<212>  DNA
<213>  Artificial sequence

<220>
<223>  Primer sequences for FLJ39501 exons amplification_ n°12_ Reverse
       sequence

<400>  26
ctatgccctc gggatcttt                                                    19


<210>  27
<211>  19
<212>  DNA
<213>  Artificial sequence

<220>
<223>  Primer sequences for FLJ39501 RT-PCR_ RT-exp1_ Forward sequence

<400>  27
ctggccctaa agcaaggac                                                    19


<210>  28
<211>  21
<212>  DNA
<213>  Artificial sequence

<220>
<223>  Primer sequences for FLJ39501 RT-PCR_ RT-exp1_ Reverse sequence

<400>  28
acaggtccca tccataccaa g                                                 21


<210>  29
<211>  19
<212>  DNA
<213>  Artificial sequence

<220>
<223>  Primer sequences for FLJ39501 RT-PCR_ RT-exp2_ Forward sequence

<400>  29
ctggccctaa agcaaggac                                                    19


<210>  30
<211>  20
<212>  DNA
<213>  Artificial sequence

<220>
<223>  Primer sequences for FLJ39501 RT-PCR_ RT-exp2_ Reverse sequence

<400>  30
agtcagatcg tcccactcca                                                20